# EUROPEAN PATENT APPLICATION

(11) **EP 2 130 547 A1**
(43) Date of publication of application: **09.12.2009**
(21) Application number: 08157799.1
(22) Date of filing: 06.06.2008
(51) Int. Cl.: A61K 38/20, C07K 14/54, A61P 29/00, A61P 37/00, A61P 1/00, A61K 39/395

(54) **IL-25 for use in the treatment of inflammatory diseases**

(71) Applicant: Giuliani International Limited, 20129 Milano (IT)
(72) Inventor: Monteleone, Giovanni, I-00133, Rome (IT)
(74) Representative: Gates, Marie Christina Esther

(57) **Abstract**

The invention in some aspects relates to inflammatory diseases resulting from cytokine production by CD14+ cells such as Inflammatory Bowel Disease and Sepsis. In some aspects the invention relates to methods for regulating the cytokine production of cells, in particular CD 14+ cells. In some aspects the invention provides methods for the treatment of inflammatory disease resulting from cytokine production by CD 14+ cells. In some aspects, the invention provides alternative medicaments for the treatment of inflammatory disease, in which CD 14+ plays a role.

## Description

### Field of the invention

The invention in some aspects relates to inflammatory diseases resulting from cytokine production by CD14+ cells such as Inflammatory Bowel Disease and Sepsis. In some aspects, the invention relates to methods for regulating the cytokine production of cells, in particular CD14+ cells. In some aspects, the invention provides methods for the treatment of inflammatory disease resulting from cytokine production by CD14+ cells. In some aspects, the invention provides alternative medicaments for the treatment of inflammatory disease associated with CD14+ cells.

### Background of the invention

A broad spectrum of diseases are associated with inflammatory cytokine response resulting from CD14+ cells. Inflammatory bowel diseases (IBD) such as Crohn's disease (CD) and ulcerative colitis are associated with poor quality of life and significant morbidity and mortality. Steroids, immunosuppressors, therapeutic compounds and anti-TNF-alpha antibodies are drugs widely used in CD 14+ cell-related pathologies, and with limited success. For example, it is reported that approximately 25% of patients undergoing steroid treatment experience steroid-dependence. Moreover, the use of certain therapeutic compounds is associated with enhanced risk of severe side effects and approximately 50% of patients are resistant to such a treatments. Thus, there remains a need for improved treatments for diseases associated with inflammatory cytokine response resulting from CD-14+ cells.

### Summary of the Invention

The invention broadly relates to methods of treating diseases associated with inflammatory cytokine response resulting from CD-14+ cells. In some aspects, the invention relates to treatment methods and/or alternative medicaments based on interleukin (IL)-25, and derivatives or fragments thereof. According to some aspects the invention relates to methods for regulating the cytokine production of cells, in particular CD14+ cells. In some aspects, the invention provides methods for the treatment of inflammatory disease resulting from cytokine production by CD14+ cells. In some aspects, the invention provides alternative medicaments or the treatment of inflammatory diseases associated with CD 14+ cells.

IL-25, a member of the IL-17 cytokine family, can both facilitate pathogenic Th2 cell responses, and inhibit Th17-mediated immune diseases. The cell types that respond to IL-25 and the mechanisms by which IL-25 differently regulates immune reactions are not however entirely known. Here we show that monocytes express high levels of IL-25 receptor, and respond to IL-25 by down-regulating the expression of inflammatory cytokines induced by various inflammatory stimuli. Inhibition of the cytokine response by IL-25 occurs via a p38-driven SOCS-3 induction-dependent mechanism. Consistently, in vivo administration of IL-25 inhibits monocyte-derived cytokines, provides protection from LPS-induced lethal endotoxemia, and largely prevents and reverses experimental colitis. Data indicate that IL-25 negatively regulates monocyte cytokine response, thus suggesting that IL-25 therapy may be useful for attenuating monocyte-associated immunity.

The current invention provides methods of regulating the cytokine production of cells comprising administering interleukin (IL)-25, a derivative of or a fragment of IL-25. These cells can be CD14+ cells. The cytokines are involved in the TH1 response and /or TH-17 response. The cytokines can be IL-6 and IL-8, TNF-α, IL12, IL-23, IL-22, IL-21 IFN-gamma, IL-17 and IL-1β. The regulation of the cytokine response of CD14+ cells by IL-25 may be dependent on SOCS-3.

The invention further provides for the use of IL-25, or a derivative of or a fragment of IL-25, in the preparation of a medicament for the treatment of disease. The disease may be an inflammatory disease in which CD 14+ cells play a part. The disease can be but is not limited to inflammatory bowel disease (IBD) such as Crohn's disease (CD) or ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis and neoplasia and other CD14+ cell related diseases.

A further embodiment of the present invention provides for a pharmaceutical composition containing a pharmaceutically active amount of an interleukin for the treatment of disease. The interleukin may be interleukin-25, a derivative of or a fragment of IL-25.

The invention further provides for a method of down regulating the inflammatory cytokine response of CD14+cells, comprising administering of interleukin-25, a derivative or a fragment thereof.

A further embodiment of the current invention provides for an antibody directed at IL-25R in the preparation of a medicament for the treatment of disease. An antibody to IL-25R, will mimic the mode of action of IL-25.

The present invention will now be described with reference to the following figures.

### BRIEF DESCRIPTION OF THE FIGURES

FIG. 1. Real-time PCR expression of the IL-25 receptor (IL-25R) in human blood CD14+ cells.
FIG. 2. Inhibition of inflammatory cytokines induced by LPS and PGN in human blood CD14+ cells by IL-25.
FIG. 3. CD80, CD86, and HLA-DRII levels in blood CD14+ cells cultured in the presence or absence of LPS, PGN, or CpG.
FIG. 4. IL-10 expression levels in CD14+ cells stimulated with IL-25.
FIG. 5. Real-time PCR data showing the expression of IL-25R in intestinal CD14+ cells and are functionally capable of responding to IL-25.
FIG. 6. IL-25 expression is down regulated in the inflamed colon of patients with IBD.
FIG. 7. IL-25 expression is down regulated in experimental models of IBD.
FIG. 8. (a) Percentage of mortality in control mice and mice with PGN-colitis treated or untreated with IL-25. (b) Macroscopic pictures of colon samples of control mice and mice with PGN-colitis treated or untreated with IL-25.
FIG. 9. (a)Percentage of mortality in control (ETOH) mice and colitic mice (TNBS-induced) left untreated or treated with IL-25. (b) Representative macroscopic pictures of colons in control (ETOH) and colitic mice either left untreated or treated with IL-25. (c) Macroscopic pictures of colon samples of control mice and mice with TNBS-colitis treated or untreated with IL-25. (d) Expression ofIL-12/p70 in mice with TNBS-colitis treated or untreated with IL-25. (e) Expression of IFN-γ in mice with TNBS-colitis treated or untreated with IL-25.
FIG. 10. IL-25 receptor (IL-25R) is expressed by human blood and mucosal CD14+ cells. (a) Representative dot-plots showing IL-25R in human PBMC. (b) Percentages of CD3, CD14, CD19, and CD56 cells positive for IL-25R in fresh blood samples. (c) CD16+ cells express IL-25R. CD14+ cells were purified from PBMC and stained for IL-25R and CD16. (d) Real-time PCR data for the membrane bound IL-25R isoform RNA transcripts in blood CD14+ (CD14) cells and The-1.
FIG. 11. (a) SOCS3 levels in response to IL-25 stimulation in blood CD14+ cells induced by LPS and PGN. (b) Cells were cultured with control or SOCS-3 siRNA. After 2 days, cells were washed and cultured with IL-25 for 30 minutes followed by stimulation with LPS for 2 hours. (c) IL-25 fails to inhibit cytokine expression induced by LPS and PGN in SOCS-3-deficient cells.

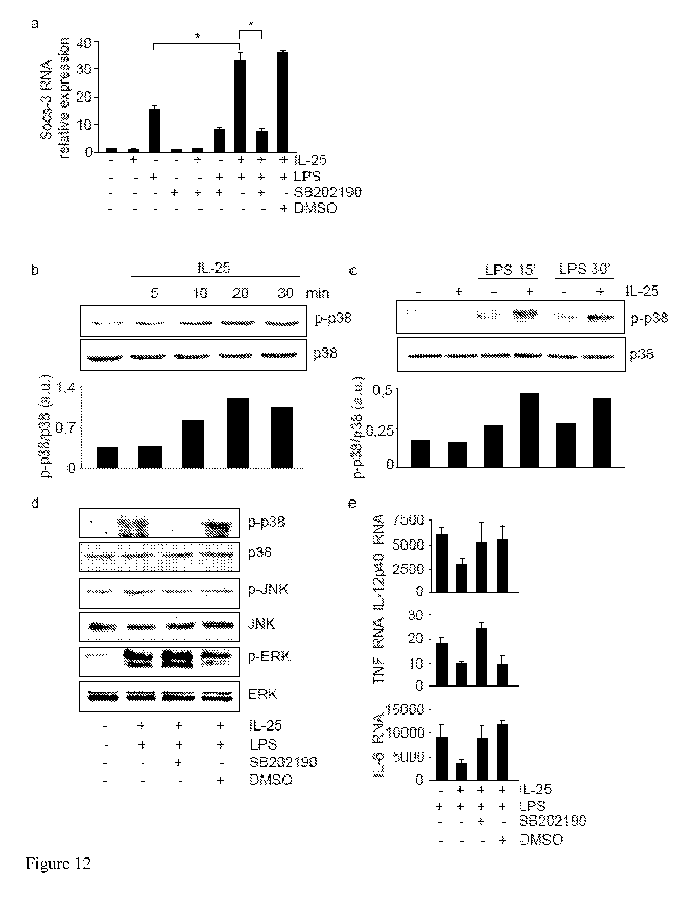

FIG. 12. (a) CD14+ cells were pre-incubated with SB202190, or DMSO then treated with IL-25 and/or LPS. (b) CD14+ cells were cultured with or without IL-25 for the indicated time points, and both p-p38 and total p38 were evaluated. (c) CD14+ cells were cultured with or without IL-25 for 30 minutes and then either left unstimulated or stimulated with LPS for the indicated time points. (d) Pre-incubation of cells with SB202190 prevents the phosphorylation of p38, but not that of both JNK and ERK1/2, in response to IL-25 and LPS stimulation. (e) Pre-incubation of cells with SB202190 prevents the negative regulation of IL-20 on the cytokine response induced by LPS.
FIG. 13. IL-25-treated mice exhibit decreased serum levels ofIL-12p70 (a), TNF-α (b), and IL-6 (c) following systemic administration of LPS or PGN. (d) IL-25 protects mice from lethality.
FIG. 14. (a) PGN colitis was induced in Balb/c mice by intrarectal administration of 50% ethanol followed by soluble PGN again per rectum. (b) Histologic evaluation of PGN colitis in mice treated or not with IL-25. Administration of IL-25 in mice with PGN-colitis results in decreased tissue expression ofIL-12p70 (c), IL-12p40 (d), and IFN-γ (e).
FIG. 15. (a) Body weight changes of control and colitic mice either untreated or treated with IL-25. (b) H&E stained sections of colon collected from mice with PGN-colitis either left untreated or treated with IL-25. (c-e) Administration of IL-25 in mice with PGN-colitis results in decreased tissue expression ofIL-12p70 (c), IL-12p40 (d), and IFN-γ (e). (f) IL-25 ameliorates the TNBS-mediated colitis. g-i. Administration of IL-25 in mice with TNBS-colitis results in decreased tissue expression ofIL-12p70 (g), IL-12p40 (h), and IFN-γ (i).
FIG. 16. Colon LPMC were isolated from mice with PGN- (a) or TNBS- (b) colitis, either receiving PBS or IL-25 the day after the induction of colitis, on day 4, and stimulated in vitro with PGN or LPS for 6 hours.
FIG. 17. Representative dot-plots showing TLR-2 and TLR-4 in blood CD14+ cells either left unstimulated (Uns) or stimulated with 50 ng/ml IL-25 in the presence or absence of PGN (a) or LPS (b) for 6 hours.
FIG. 18. IL-25 does not alter the ability of LPS-stimulated human blood CD14+ cells to phagocyte fluorescent latex beads.
FIG. 19. IL-25 inhibits the expression of inflammatory cytokines induced by TNF-α and IFN-γ in human blood CD14+ cells.
FIG. 20. SOCS-1 RNA levels in response to IL-25 stimulation in blood CD14+ cells induced by LPS and PGN.
FIG. 21. Induction of SOCS-3 by IL-25 does not rely on JNK/ERK activity. CD14+ cells were preincubated with 420116 (a JNK inhibitor), PD9805 (an ERK inhibitor), or vehicle (DMSO) then treated with IL-25 and/or LPS as indicated in materials and methods.
FIG. 22. Induction of SOCS-3 by IL-25 relies on p38 Map kinase activity. CD14+ cells were preincubated with SB202190 (a p38 Map kinase inhibitor) (panel a), 420116 (a JNK inhibitor) (panel b), PD9805 (an ERK inhibitor) (panel b), or vehicle (DMSO) then treated with IL-25 and/or PGN as indicated in materials and methods.
FIG. 23. IL-25 inhibits the expression of TNF-α (a) and IL-6 (b) induced by LPS and PGN in CD 14+ cells isolated from the spleen of Balb/c mice.

### DETAILED DESCRIPTION OF THE INVENTION

The invention in some aspects provides methods of treating inflammatory diseases, also referred to herein as inflammatory disorders. In some aspects the invention relates to methods for regulating the cytokine production of cells, in particular CD 14+ cells. In some aspects, the invention provides a methods for the treatment of inflammatory disease resulting from cytokine production by CD14+ cells. In some aspects, the invention provides alternative medicaments for the treatment of inflammatory disease, in which CD 14+ plays a role. Thus, the invention, in some aspects, is relevant to the treatment of a variety of diseases, for example, inflammatory bowel disease, Crohn's disease (CD), ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis and neoplasia and other CD14+ cell related diseases.

### Inflammatory Diseases

According to some aspects, the invention relates to the treatment of inflammatory diseases associated with CD14+ cells. In humans, CD14 is a single-copy gene encoding 2 protein forms: a 50- to 55-kD glycosylphosphatidylinositol-anchored membrane protein (mCD 14) and a monocyte or liver-derived soluble serum protein (sCD 14) that lacks the anchor. Both molecules are critical for lipopolysaccharide (LPS)-dependent signal transduction, and sCD 14 confers LPS sensitivity to cells lacking mCD 14. In some cases, increased CD 14 levels are associated with inflammatory diseases and high mortality in gram-negative bacterial shock.

The treatment methods of the present invention are relevant to inflammatory diseases resulting from cytokine production by CD14+ cells. Exemplary inflammatory diseases resulting from cytokine production by CD 14+ cells include inflammatory bowel disease (IBD) such as Crohn's disease (CD) or ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis and neoplasia and other CD14+ cell related diseases. In some preferred embodiments, the treatment methods are useful for inflammatory bowel disease (IBD) or sepsis.

Inflammatory bowel disease (IBD) is a group of inflammatory conditions of the large intestine and/or small intestine. IBD represent chronic tissue-destructive diseases, the etiology of which remains unknown. The main forms of IBD are Crohn's disease (CD) and ulcerative colitis. IBD is characterized by an infiltration of CD 14+ cells to the intestinal mucosa. Evidence suggests that CD14+ cells play a role in the tissue-damaging immune response, due to their ability to produce huge amounts of inflammatory cytokines. In relation to Crohn's disease, mucosal CD14+ cells synthesize high levels of Interleukin (IL)-12 and IL-23. These cytokines are involved in the control of the Th1 and Th17 cell response, respectively. These responses in turn, result in the production of IFN-gamma and IL-17 and IL-22, respectively. In CD and ulcerative colitis, mucosal CD14+ cells are also a source of tissue necrosis factor (TNF)-α, IL-1β, IL-6 and IL-8. The molecular mechanisms underlying the enhanced production of inflammatory cytokines by CD 14 cells in IBD is not fully understood.

Individuals with IBD may present with any of the following exemplary symptoms: abdominal pain, vomiting, diarrhea, hematochezia, weight loss and various associated complaints or diseases (arthritis, pyoderma gangrenosum, primary sclerosing cholangitis). Diagnosis is often by colonoscopy with biopsy of pathological lesions. In some embodiments, diagnosis includes a determination of CD14 expression levels in disease associated cells (e.g., cells obtained from biopsy of pathological lesions (clinical samples)). Methods for determining CD14 expression levels are known in the art and disclosed herein (e.g., see examples).

In general, steroids, immunosuppressors, and anti-TNF-alpha antibodies are drugs widely used in CD14+ cell-related pathologies (e.g., psoriasis, vasculitis, rheumatoid arthritis). The therapeutic intervention for patients with Crohn's disease and patients with ulcerative colitis is markedly influenced by the type and severity of the clinical symptoms, the type and the extension of the lesions, as well as by the presence of any complications. In general, salicylazosulfapyridine and 5-aminosalicylic acid are drugs of proven efficacy in the management of patients with mild IBD and for prolonging remission of the disease. Moderate-severe IBD cases require the use of cortico-steroids and clinical remission is obtainable in two thirds of patients receiving such a treatment. However, it is reported that approximately 25% of patients undergoing such treatment experience steroid-dependence. Moreover, chronic administration of steroids is associated with a high risk of adverse side effects. Therefore, these subgroups of patients who do not respond well or cannot tolerate steroid treatment are candidates for treatment with immunosuppressors such as azathioprine, 6-mercaptopurine, cyclosporine, and methotrexate or biological drugs for example anti-TNF-α antibodies. In general however, the use of these compounds is also associated with enhanced risk of severe side effects and approximately 50% of patients are resistant to such a treatment.

Usually the treatment is started by administering drugs with high anti-inflammatory affects, such as Prednisone. Once the inflammation is successfully controlled, the patient is usually switched to a lighter drug to keep the disease in remission, such as Asacol, a mesalamine. If unsuccessful, a combination of the aforementioned immunosurpression drugs with a mesalamine (which may also have an anti-inflammatory effect) may or may not be administered, depending on the patient.

As discussed herein, depending on a number of factors (e.g., the level of severity), IBD may require immunosuppression to control the symptoms. such as azathioprine, methotrexate, or 6-mercaptopurine. Commonly, treatment of IBD requires a form of mesalamine. In use for several years in Crohn's disease patients and recently in patients with Ulcerative Colitis, biologicals (biologics) such as the intravenously administered Remicade have been used. Severe cases may require surgery, such as bowel resection, strictureplasty or a temporary or permanent colostomy or ileostomy. Alternative medicine treatments for bowel disease exist in various forms and are known in the art, however such methods concentrate on controlling underlying pathology in order to avoid prolonged steroidal exposure or surgical excisement. The treatment methods disclosed herein are useful in combination with any of these IBD treatment and/or surgical procedures.

In some aspects, the invention provides methods for the treatment of sepsis. Sepsis, or endotoxemia, as used interchangeably herein, refer to a systemic inflammatory response to an infection (e.g., bacterial, viral, fungal, or parasitic infection). Bacterial sepsis can be caused by both gram-positive and gram-negative bacteria, the latter primarily due to the bacterial endotoxin liposaccaride (LPS). In addition, bacterial sepsis can be induced by a variety of bacteria, for example Pseudomonas aeriginosa, Escherichia coli, Proteus, Klebsiella, Enterobacter and Serratia. However, sepsis is not limited to these specific bacterial causes and other exemplary bacterial pathogens will be apparent to one skilled in the art. Sepsis also includes septic shock (e.g., bacterial shock), and any disease or disorder related to or resulting from septic shock, including, for example, hypotension, oliguria, tachycardia, tachypnea, and fever.

In some cases, the signs and symptoms of severe sepsis may be subtle. Sepsis is considered a systemic inflammatory response syndrome (SIRS) resulting from infection (bacterial, viral, fungal, or parasitic), and is associated with a continuum of events which may include severe sepsis, septic shock, multiple organ dysfunction syndrome (MODS). SIRS typically includes, but is not limited to, the presence of more than one of the following manifestations: temperature ≥38°C or ≤36°C (≥100.4°F or ≤96.8°F), Heart rate ≥90 beats/min, Tachypnea, as manifested by a respiratory rate ≥20 breaths/min or hyperventilation, as indicated by a PaCO2 ≤32 mmHg, alteration of white blood cell count ≥12,000 cells/mm3, ≤4,000 cells/mm3, or the presence of >10% immature neutrophils. Other clinical manifestation may indicative of SIRS and, therefore, sepsis in some cases. In some cases, sepsis is determined by detecting pathogens in a clinical sample (e.g., by culture, stain, or polymerase chain reaction(PCR)).

### IL-25

The invention further provides for the use of IL-25, or a derivative of or a functional fragment of IL-25, in the preparation of a medicament for the treatment of disease. Interleukin (IL)-25, also known as IL-17E, is a member of the structurally related IL-17 cytokine family. Its in vivo and in vitro biological functions are markedly different from other IL-17 family members however. IL-17 is expressed by activated CD4⁺ T-cells and mediates the induction of pro-inflammatory cytokines such as IL- 10 and IL-6 and TNFα. IL-25 on the other-hand is expressed in activated Th2 T-cells and in non-T cells. Transgenic expression of IL-25 and systemic administration of the recombinant cytokine in mice has been shown to induce a Th-2 cell-associated mucosal inflammatory pathologies. A Th-2 response is characterized by epithelial cell hyperplasia, increased mucous production, and recruitment of inflammatory cells into inflamed tissues with elevated expression of IL-4 and IL-5 and IL-13. In contrast, studies in murine models of autoimmunity have shown that IL-25 can negatively regulate the development and/or amplification of Th17-mediated pathology. Therefore, it has been shown that IL-25 can either promote or inhibit specific inflammatory responses. Literature also suggests that IL-25 has a role in promoting allergic responses. However, the active role IL-25 plays in the regulation of immunity and infection induced inflammation in vivo remains largely unknown. In addition, recent work indicates that the IL-25 gene has been identified in a chromosomal region associated with autoimmune diseases of the gut such as inflammatory bowel disease (IBD), although, prior to the present invention, no direct evidence suggests that IL-25 plays any role in this disease (Buning et al., The interleukin-25 gene located in the inflammatory bowel disease (IBD) 4 region: no association with inflammatory bowel disease. European Journal of Immunogenetics, 2003, Volume 30, pages 329-333).

The biological functions of IL-25 are mediated by a receptor called IL-25R. IL-25R was first identified as an IL-17B receptor (IL-17BR), also called IL-17 receptor homolog1 and EVI27. IL-25R is a single pass transmembrane receptor that is expressed in the kidneys, liver, intestine and on the surface of human basophils and dendritic cells that have been activated in the presence of Th2-associated stimuli.

A previous study by Fort MM et al, disclosed the presence of IL-25 RNA in the gastrointestinal tract of mice. However, the association of IL-25 with inflammatory bowel disease in mice or humans has never been proven. WO2006088833 recently disclosed polypeptides related to the IL-17 signaling pathway. In particular, it reported the potential use for signal antagonists of the IL-17F signaling pathway in methods of treating diseases related to IL-17F signaling, such as inflammatory diseases. EP1863520 disclosed the use of IL-25 to inhibit tumor growth. The anti-tumor effect of IL-25 described in EP1863520 is attributed to increased infiltration into the tumor site. WO200409684 disclosed a number of peptides including IL-25, linked to a carrier protein to form a vaccine compound. The described vaccine functions to reduce allergic responses, induced by over-expressed cytokines by way of the induction of auto-antibodies that bind to cytokines and thus prevent the allergic response.

As discussed herein, there is for a more effective mode of treatment for inflammatory diseases with reduced risk of the severe side effects associated with the current methods of treatment. The current invention provides the use of IL-25 as an alternative medicament for the treatment of inflammatory diseases.

As used herein IL-25 refers to refers to full-length protein, functional homologues, functional fragments, derivatives and variants thereof. Functional homologues and functional fragments are segments or arrangements of segments of the full length protein that have substantially the same activity as full-length IL-25 with respect to the inhibition of inflammatory responses (e.g., inflammatory cytokine responses) resulting from cytokine production by CD14+ cells. In some embodiments, functional homologues and functional fragments are segments or arrangements of segments of the full length protein have substantially the same activity as full-length IL-25 with respect to the inhibition of inflammatory responses resulting from cytokine production by CD 14+ cells **and that contain one or more conserved functional domains???. [PLEASE PROVIDE DETAILS ON CONSERVED FUNCTIONAL DOMAINS].**

In some embodiments, functional variants are full-length proteins, functional homologues, or functional fragments, with amino acid additions, deletions, substitutions, and/or non-naturally occurring amino-acids that have substantially the same activity as full-length IL-25 with respect to the inhibition of inflammatory responses resulting from cytokine production by CD14+ cells. In some embodiments, functional variants are full-length proteins, functional homologues, or functional fragments, with one or more conservative substitutions that have substantially the same activity as full-length IL-25 with respect to the inhibition of inflammatory responses resulting from cytokine production by CD 14+ cells. As used herein, "conservative substitution" refers to an amino acid substitution which does not alter the relative charge or size characteristics of the peptide in which the amino acid substitution is made. Conservative substitutions of amino acids include substitutions made among amino acids with the following groups: (1) M, I, L,V; (2) F,Y,W; (3) K,R,H; (4) A,G; (5) S,T; (6) Q,N; and, (7) E,D. Such substitutions can be made by a variety of methods known to one of ordinary skill in the art to IL-25, and functional fragments thereof, of the invention. These and other methods are known to those of ordinary skill in the art and may be found in references which compile such methods, e.g. Sambrook. et al., Molecular Cloning: A Laboratory Manual, 2nd edition, Cold Spring Harbor Laboratory Press, 1989. The activity of functional fragments of IL-25 of the invention can be tested by binding and activity assays known in the art and disclosed herein (See Examples).

In some cases it may be desirable to construct a fusion protein with IL-25 full-length protein, functional homologues, functional fragments, or variants thereof. Appropriate fusion partner proteins will be apparent to the skilled artisan. In some cases, a fusion partner protein may be useful to protect IL-25 against proteolytic degradation *in vivo*. Proteases and other proteolytic enzymes are ubiquitous, particularly in the gastro-intestinal tract, and therefore peptides are usually susceptible to degradation in multiple sites upon administration, and to some extent in the blood, the liver, the kidney, and the vascular endothelia. Fusion proteins that, among other things, protect against degradation, are well known in the art, for example as described in Prokai, 1997, Exp. Opin. Ther. Patents 7:233-245, Tamai et al., 1996, Adv. Drug Delivery Rev. 19: 401-424 and Zhou et al., 1991, Int. J. Pharm. 75: 97-115; Zhou et al., 1991, Int. J. Pharm. 75: 97-115. In one example, WO 98/22577 discloses a method for increasing the resistance of a "core protein" to proteolytic degradation by linking it to a "stabilizing polypeptide". In another example, WO 97/24445 discloses fusion proteins of albumin and therapeutic proteins or variants thereof. In yet another example, W098/28427 discloses an Fc-fusion protein, wherein the Fc portion confers stability and can be conjugated directly or via a peptide linker. Fusion proteins can be prepared using methods well known in the art including recombinant DNA methods and chemical cross-linking methods.

In some cases, IL-25 agonists can be small molecules and/or peptide mimetics that inhibit inflammatory responses resulting from cytokine production by CD 14+ cells such as those disclosed herein (e.g., IBD, sepsis). One of skill in the art can identify such small molecules and/or peptide mimetics by screening the ability of the small molecules and/or peptide mimetics to inhibit inflammatory response in an *in vitro* or *in vivo* assay such as those disclosed herein. The activity of the agonist can be defined in terms of its ability to inhibit an inflammatory response resulting from cytokine production by CD 14+ cells. Exemplary assays for measuring this activity are provided herein (e.g., see Examples) and will be known to one of ordinary skill in the art.

According to some aspects, the invention provide agonistic IL-25 receptor binding molecules. In some embodiments, binding molecules useful according to the invention are antibodies or functionally active antibody fragments. In preferred embodiments, binding molecules useful according to the invention are antibodies or functionally active antibody fragments that bind and activate the IL-25 receptor. Antibodies are well known to those of ordinary skill in the science of immunology. Many of the binding molecules described herein are available from commercial sources as intact functional antibodies, as described above. As used herein, the term "antibody" means not only intact antibody molecules but also fragments of antibody molecules retaining specific binding ability. Such fragments are also well known in the art and are regularly employed both *in vitro* and *in vivo*. In particular, as used herein, the term "antibody" means not only intact immunoglobulin molecules but also the well-known active fragments F(ab')₂, and Fab. F(ab')₂, and Fab fragments which lack the Fc fragment of intact antibody, clear more rapidly from the circulation, and may have less non-specific tissue binding of an intact antibody (Wahl et al., J. Nucl. Med. 24: 316-325 (1983*)).*

As is well-known in the art, the complementarity determining regions (CDRs) of an antibody are the portions of the antibody which are largely responsible for antibody specificity. The CDR's directly interact with the epitope of the antigen (see, in general, *Clark, 1986; Roitt, 1991*)*.* In both the heavy chain and the light chain variable regions of IgG immunoglobulins, there are four framework regions (FR1 through FR4) separated respectively by three complementarity determining regions (CDR1 through CDR3). The framework regions (FRs) maintain the tertiary structure of the paratope, which is the portion of the antibody which is involved in the interaction with the antigen. The CDRs, and in particular the CDR3 regions, and more particularly the heavy chain CDR3 contribute to antibody specificity. Because these CDR regions and in particular the CDR3 region confer antigen specificity on the antibody these regions may be incorporated into other antibodies or peptides to confer the identical specificity onto that antibody or molecule.

According to one embodiment, the molecule is an intact soluble monoclonal antibody in an isolated form or in a pharmaceutical preparation. An intact soluble monoclonal antibody, as is well known in the art, is an assembly of polypeptide chains linked by disulfide bridges. Two principle polypeptide chains, referred to as the light chain and heavy chain, make up all major structural classes (isotypes) of antibody. Both heavy chains and light chains are further divided into subregions referred to as variable regions and constant regions. As used herein the term "monoclonal antibody" refers to a homogenous population of immunoglobulins which specifically bind to an epitope (i.e. antigenic determinant), e.g., of IL-25R.

The molecule useful according to the methods of the present invention may be an intact humanized a monoclonal antibody. A "humanized monoclonal antibody" as used herein is a human monoclonal antibody or functionally active fragment thereof having human constant regions and a binding CDR3 region from a mammal of a species other than a human. Humanized monoclonal antibodies may be made by any method known in the art. Humanized monoclonal antibodies, for example, may be constructed by replacing the non-CDR regions of a non-human mammalian antibody with similar regions of human antibodies while retaining the epitopic specificity of the original antibody. For example, non-human CDRs and optionally some of the framework regions may be covalently joined to human FR and/or Fc/pFc' regions to produce a functional antibody. There are commercial entities which will synthesize humanized antibodies from specific murine antibody regions commercially, such as Protein Design Labs (Mountain View California, USA).

European Patent Application 0239400, the entire contents of which is hereby incorporated by reference, provides an exemplary teaching of the production and use of humanized monoclonal antibodies in which at least the CDR portion of a murine (or other non-human mammal) antibody is included in the humanized antibody. Briefly, the following methods are useful for constructing a humanized CDR monoclonal antibody including at least a portion of a mouse CDR. A first replicable expression vector including a suitable promoter operably linked to a DNA sequence encoding at least a variable domain of an Ig heavy or light chain and the variable domain comprising framework regions from a human antibody and a CDR region of a murine antibody is prepared. Optionally a second replicable expression vector is prepared which includes a suitable promoter operably linked to a DNA sequence encoding at least the variable domain of a complementary human Ig light or heavy chain respectively. A cell line is then transformed with the vectors. Preferably the cell line is an immortalized mammalian cell line of lymphoid origin, such as a myeloma, hybridoma, trioma, or quadroma cell line, or is a normal lymphoid cell which has been immortalized by transformation with a virus. The transformed cell line is then cultured under conditions known to those of skill in the art to produce the humanized antibody.

As set forth in European Patent Application 0239400 several techniques are well known in the art for creating the particular antibody domains to be inserted into the replicable vector. For example, the DNA sequence encoding the domain may be prepared by oligonucleotide synthesis. Alternatively a synthetic gene lacking the CDR regions in which four framework regions are fused together with suitable restriction sites at the junctions, such that double stranded synthetic or restricted subcloned CDR cassettes with sticky ends could be ligated at the junctions of the framework regions. Another method involves the preparation of the DNA sequence encoding the variable CDR containing domain by oligonucleotide site-directed mutagenesis. Each of these methods is well known in the art. Therefore, those skilled in the art may construct humanized antibodies containing a murine CDR region without destroying the specificity of the antibody for its epitope.

Human monoclonal antibodies may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, new York, 1987), and Boerner et al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immuno., 7:33 (1993) and US Patent No. 5,569,825 issued to Lonberg).

The binding peptides may also be functionally active antibody fragments. Significantly, as is well-known in the art, only a small portion of an antibody molecule, the paratope, is involved in the binding of the antibody to its epitope (see, in general, Clark, W.R. (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions of the antibody, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd (heavy chain variable region). The Fd fragments are the major determinant of antibody specificity (a single Fd fragment may be associated with up to ten different light chains without altering antibody specificity) and Fd fragments retain epitope-binding ability in isolation.

The terms Fab, Fc, pFc', F(ab')₂ and Fv are used consistently with their standard immunological meanings [Klein, Immunology (John Wiley, New York, NY, 1982); Clark, W.R. (1986) The Experimental Foundations of Modern Immunology (Wiley & Sons, Inc., New York); Roitt, I. (1991) Essential Immunology, 7th Ed., (Blackwell Scientific Publications, Oxford)].

### TREATMENT

In some aspects, the invention provides methods for treating an individual having, suspected of having, or at-risk of having an inflammatory disorder associated. As used herein, a individual is a mammal, including but not limited to a dog, cat, horse, cow, pig, sheep, goat, chicken, rodent, or primate. Individuals can be house pets (e.g., dogs, cats), agricultural stock animals (e.g., cows, horses, pigs, chickens, etc.), laboratory animals (e.g., mice, rats, rabbits, etc.), zoo animals (e.g., lions, giraffes, etc.), but are not so limited. In some embodiments, an individual is an animal model (e.g., ulcerative colitis mouse model, endotoxemia mouse model). Preferred individuals are humans (human subjects). The human subject may be a pediatric or adult subject. In some embodiments the adult subject is a geriatric subject. An individual (subject) that is under the care of a physician may be referred to in some cases as a patient.

As used herein treatment, or treating, includes amelioration, cure or maintenance (i.e., the prevention of relapse) of a disease. Treatment after a disease has started aims to reduce, ameliorate or altogether eliminate the disease, and/or its associated symptoms, to prevent it from becoming worse, or to prevent the disease from re-occurring once it has been initially eliminated (i.e., to prevent a relapse). Treatment before a disease has started (e.g., a prophylactic treatment) typically aims to prevent the disease from occurring.

To determine whether a individual is afflicted with an inflammatory disorder of the invention (e.g., disorder resulting from cytokine production by CD14+ cells such as IBD, UC, and sepsis), a clinical sample may be obtained from the individual (e.g., for diagnostic purposes). A clinical sample can be any biological specimen (e.g., a blood sample) useful for determining the presence of (e.g., diagnosing) an inflammatory disorder of the invention. Exemplary, biological specimens include an isolated cell, an isolated tissue (e.g., biopsy), saliva, gingival secretions, cerebrospinal fluid (spinal fluid), gastrointestinal fluid, mucus, urogenital secretions, synovial fluid, blood, serum, plasma, urine, cystic fluid, lymph fluid, ascites, pleural effusion, interstitial fluid, intracellular fluid, ocular fluids, seminal fluid, mammary secretions, vitreal fluid, and nasal secretions. In preferred embodiments, a clinical sample is a blood (plasma) sample. Typically, clinical samples (e.g., for diagnostic purposes) are obtain from individuals (e.g., patients).

In one aspect, the invention provides a method for preventing an inflammatory disorder resulting from cytokine production by CD14+ cells in an individual by administering to the individual IL-25, or a functional fragment thereof. Individuals at risk for an inflammatory disorder resulting from cytokine production by CD14+ cells can be identified, for example, by any or a combination of the diagnostic or prognostic assays known in the art and described herein. Administration of a preventative treatment (prophylactic treatment) can occur prior to the manifestation of symptoms characteristic of an inflammatory disorder resulting from cytokine production by CD14+ cells.

Another aspect of the invention provides to methods for treating an individual having, or suspected of having, an inflammatory disorder resulting from cytokine production by CD14+ cells. These methods involve administering to an individual IL-25, or a functional fragment thereof. Administration of IL-25, or a functional fragment thereof, to a subject for the treatment or prevention of inflammatory disorders may be alone or in combination with other agents known to aid in the treatment or prevention of inflammatory disorders. Administration of IL-25, or a functional fragment thereof and another agent (e.g., steriod, antibiotic) may be serially or as a mixture. In some cases, an IL-25 agonist, or an IL-25 mimetic, e.g., a small molecule, may supplant or complement IL-25 as therapy for inflammatory disorders.

In one aspect, the invention provides a method for preventing IBD (e.g., Crohn's diease, Ulcerative Colitis) in an individual by administering to the subject IL-25, or a functional fragment thereof. Individuals at risk for IBD can be identified by, for example, any or a combination of the diagnostic or prognostic assays known in the art and described herein. Administration of a prophylactic treatment can occur prior to the manifestation of symptoms characteristic of IBD, e.g., prior to a procedure, e.g., a surgical procedure, which places an individual at risk for IBD, such that IBD is prevented or, alternatively, delayed in its progression.

Another aspect of the invention provides to methods for treating an individual having, or suspected of having, IBD. These methods involve administering to an individual IL-25, or a functional fragment thereof. Administration of IL-25, or a functional fragment thereof, to a subject for the treatment or prevention of IBD may be alone or in combination with other agents known to aid in the treatment or prevention of IBD, e.g., anti-inflammatory agents, antibiotics, anti-TNF-α antibodies, and/or anti-LPS antibodies, which may be produced as described in U.S. Pat. No. 6,315,999, the contents of which are incorporated herein by reference). Administration of IL-25, or a functional fragment thereof and another agent (antibiotic) for the treatment of IBD may be serially or as a mixture.

In one aspect, the invention provides a method for preventing sepsis in an individual by administering to the subject IL-25, or a functional fragment thereof. Individuals at risk for sepsis can be identified by, for example, any or a combination of the diagnostic or prognostic assays known in the art and described herein. Administration of a prophylactic treatment can occur prior to the manifestation of symptoms characteristic of sepsis, e.g., prior to a procedure, e.g., a surgical procedure, which places an individual at risk for sepsis, such that sepsis is prevented or, alternatively, delayed in its progression. For example, the methods and compositions of the invention can be used to prevent sepsis in an individual at-risk of sepsis. There are a variety of situations known in the art in which an individual may be deemed at-risk of sepsis, for example, immunocompromised individuals, patients receiving cancer therapy, low birth weight infants, intra-abdominal surgery patients, individuals having severe urinary tract infection, intensive care unit patients receiving anti-infective agents, individuals having severe community-acquired pneumonia (CAP), burn and trauma victims, individuals having HIV/AIDS, Meningitis, or Cellulitis, hospitalized patients receiving cytotoxic and immunosuppressive agents and individuals having chronic diseases including diabetes, heart failure, chronic renal failure, and hepatitis. These examples are not meant to be limiting and the prophylactic treatment methods of the invention may be useful in any instance where an individual is at risk of sepsis.

Another aspect of the invention provides methods for treating an individual having, or suspected of having, sepsis. These methods involve administering to an individual IL-25, or a functional fragment thereof. Administration of IL-25, or a functional fragment thereof, to a subject for the treatment or prevention of sepsis may be alone or in combination with other agents known to aid in the treatment or prevention of sepsis, e.g., antibiotics, anti-TNF-α antibodies, and/or anti-LPS antibodies. In another embodiment, IL-25, or a functional fragment thereof, may be administered in combination with any agent which acts as a protease inhibitor to inhibit the complement system, e.g., through inhibition of C1, C1s, or C1r, and/or any agent which inhibits contact system activation, e.g., through inhibition of plasma kallikrein, factor XIa, or factor XIIa, for example. Administration of IL-25, or a functional fragment thereof and another agent (antibiotic) for the treatment of sepsis may be serially or as a mixture.

In some embodiments, the treatment comprises administering to an individual having, or at risk of having, an inflammatory disorder resulting from cytokine production by CD14+ cells a therapeutically effective amount of IL-25, or a functional fragment thereof, in combination with a standard antibiotic drug. Exemplary antibiotics include, but are not limited to: Aminoglycosides, such as Amikacin, Gentamicin, Kanamycin, Neomycin, Netilmicin, Streptomycin, Tobramycin, Paromomycin; Ansamycins such as Geldanamycin and Herbimycin; the Carbacephem, Loracarbef; Carbapenems such as Ertapenem, Doripenem, Imipenem, and Meropenem; Cephalosporins, such as Cefadroxil, Cefazolin, Cefalotin, Cefalexin, Cefaclor, Cefamandole, Cefoxitin, Cefprozil, Cefuroxime, Cefixime, Cefdinir, Cefditoren, Cefoperazone, Cefotaxime, Cefpodoxime, Ceftazidime, Ceftibuten, Ceftizoxime, Ceftriaxone, Cefdinir, Cefotetan, and Cefepime; Glycopeptides such as Teicoplanin and Vancomycin; Macrolides such as Azithromycin, Clarithromycin, Dirithromycin, Erythromycin, Roxithromycin, Troleandomycin, Telithromycin, and Spectinomycin; Monobactams such as Aztreonam; Penicillins such as Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Penicillin G, Piperacillin, and Ticarcillin; Polypeptides such as Bacitracin, Colistin and Polymyxin B; Quinolones and Fluoroquinolones such as Ciprofloxacin, Enoxacin, Gatifloxacin, Levofloxacin, Lomefloxacin, Moxifloxacin, Norfloxacin, Ofloxacin, and Trovafloxacin; Sulfonamides such as, Mafenide, Prontosil, Sulfacetamide, Sulfamethizole, Sulfanilimide, Sulfasalazine, Sulfisoxazole, Sulfadiazine, Trimethoprim, and Trimethoprim-Sulfamethoxazole; Tetracyclines such as Demeclocycline, Doxycycline, Minocycline, Oxytetracycline, and Tetracycline; and others including Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampicin (Rifampin), and Tinidazole.

According to some aspects of the invention, methods for activating or enhancing the activity of SOCS-3 signaling are provided. In some aspects, activating or enhancing the activity of SOCS-3 signaling is useful to inhibit inflammatory responses resulting from cytokine production by CD14+ cells. There are a variety of ways by which SOCS-3 can be activated, or by which SOCS-3 activity can be enhanced to inhibit an inflammatory response. For example, IL-4 has been shown to enhance SOCS-3 activity (Ratthé C, Girard D. Br J Haematol. 2008 Jan;140(1):59-70). Accordingly, in some embodiments, IL-4 may be administered to an individual to treat a disease associated with inflammatory cytokine production by CD14+ cells. In some preferred embodiments, SOCS-3 activity is enhanced in a cell by contacting the cell with IL-25. In other embodiments, SOCS-3 activity is enhanced in a cell by contacting the cell with IL-4 and IL-25.

As used herein, gene therapy is a therapy focused on treating diseases, such as an inflammatory disorder, by the delivery of one or more expression vectors encoding therapeutic gene products, including polypeptides or RNA molecules, to disease associated cells. Methods for construction and delivery of expression vectors are known to one of ordinary skill in the art. One embodiment herein contemplates the use of gene therapy to deliver one or more expression vectors encoding one or more SOCS-3 Activators (e.g., IL-4, IL-25 and/or SOCS-3), to inhibit or prevent an inflammatory disorder resulting from cytokine production by CD14+ cells, and/or to treat a individual in need thereof.

### {PLEASE PROVIDE ANY OTHER METHODS OF STIMULATING SOCS-3???}

### Pharmaceutical Compositions

According to the methods of the invention, the compositions may be administered in a pharmaceutically acceptable composition. In general, pharmaceutically-acceptable carriers for proteins, binding peptides and structurally-related small molecules are well-known to those of ordinary skill in the art. As used herein, a pharmaceutically-acceptable carrier means a non-toxic material that does not interfere with the effectiveness of the biological activity of the active ingredients, i.e., the ability of IL-25 to inhibit inflammatory response in CD-14+ cells. Pharmaceutically acceptable carriers include diluents, fillers, salts, buffers, stabilizers, solubilizers and other materials which are well-known in the art. Exemplary pharmaceutically acceptable carriers for peptides in particular are described in U.S. Patent No. 5,211,657. The receptor antagonist peptides of the invention may be formulated into preparations in solid, semi-solid, liquid or gaseous forms such as tablets, capsules, powders, granules, ointments, solutions, depositories, inhalants (e.g., aerosols) and injections, and usual ways for oral, parenteral or surgical administration. The invention also embraces locally administering the compositions of the invention, including as implants.

According to the methods of the invention the compositions can be administered by injection by gradual infusion over time or by any other medically acceptable mode. The administration may, for example, be intravenous, intraperitoneal, intramuscular, intracavity, subcutaneous or transdermal. Preparations for parenteral administration includes sterile aqueous or nonaqueous solutions, suspensions and emulsions. Examples of nonaqueous solvents are propylene glycol, polyethylene glycol, vegetable oil such as olive oil, an injectable organic esters such as ethyloliate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers, (such as those based on Ringer's dextrose), and the like. Preservatives and other additives may also be present such as, for example, antimicrobials, antioxidants, chelating agents, and inert gases and the like. Those of skill in the art can readily determine the various parameters for preparing these alternative pharmaceutical compositions without resort to undue experimentation. When the compositions of the invention are administered for the treatment of pulmonary disorders the compositions may be delivered for example by aerosol.

The compositions of the invention are administered in pharmaceutically active amount (also referred to as therapeutically effective amounts). As used herein, an "effective amount" of the inhibitor of the invention is a dosage which is sufficient to inhibit the increase in, maintain or even reduce the amount of undesirable inflammatory response. Generally, a therapeutically effective amount may vary with the subject's age, condition, and sex, as well as the extent of the disease in the subject and can be determined by one of skill in the art. The dosage may be adjusted by the individual physician or veterinarian in the event of any complication. A therapeutically effective amount typically will vary from about 0.01 mg/kg to about 500 mg/kg, were typically from about 0.1 mg/kg to about 200 mg/kg, and often from about 0.2 mg/kg to about 20 mg/kg, in one or more dose administrations daily, for one or several days (depending of course of the mode of administration and the factors discussed above). In some embodiments, a therapeutically effective amount is between about 0.01 and about 1 mg/kg. In some embodiments, a therapeutically effective amount is between about 0.05 and about 0.5 mg/kg.

One of skill in the art can determine an effective amount of IL-25, or functional fragment thereof, or other therapeutic molecule (e.g., IL-25 small molecule agonist, IL-25 mimetic, IL-25R binding peptide) by screening the ability of the therapeutic molecule to inhibit inflammatory response in an in vitro or in vivo assay such as those disclosed herein. The activity of the agonist can be defined in terms of the ability of the agonist to inhibit an inflammatory response associated with CD-14+ cells. Exemplary assays for measuring this activity are provided in the Examples and has been discussed herein. For example, the exemplary assay is predictive of the ability of an IL-25 receptor agonist to inhibit an inflammatory response (e.g., IBD, sepsis) in vivo and, hence, can be used to select such receptor agonists for therapeutic applications.

### EXAMPLE 1:

### IL-25R expression in CD14+ cells

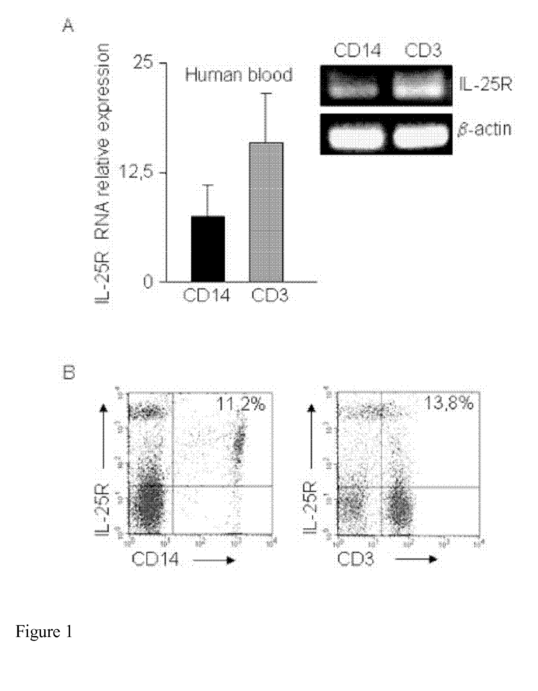

The expression of IL-25R was analyzed in blood CD14+ and CD3+ cells by Real-Time PCR. The transcripts for both the membrane-bound and soluble IL-25R isoforms were constitutively expressed in blood CD14+ cells (FIG. 1A). Flow-cytometry analysis confirmed that CD14+ cells express the cell surface-associated IL-25R.

### Response of CD14+ to stimuli

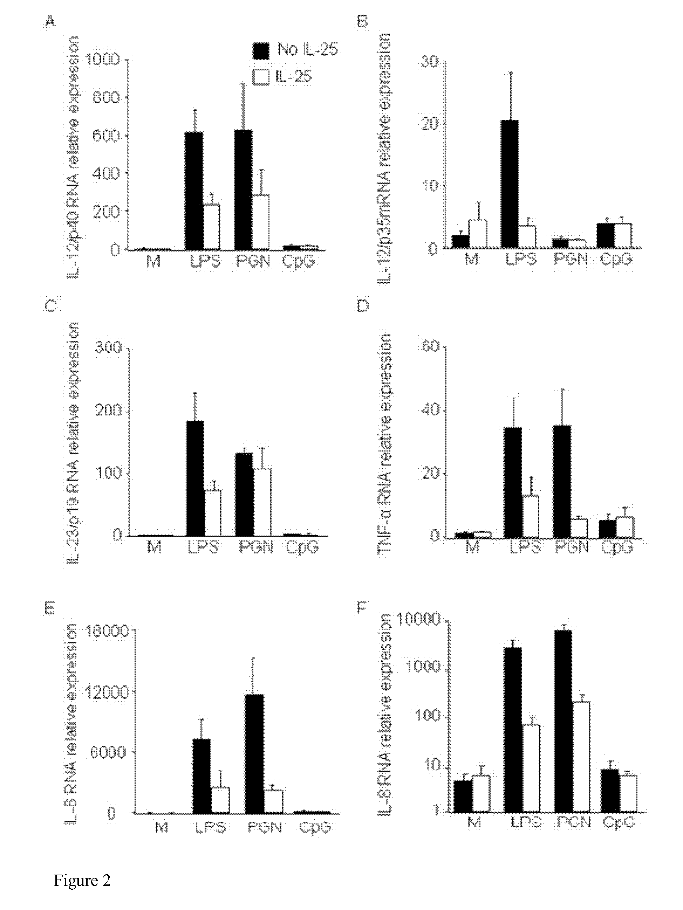

CD14+ cells were cultured with or without IL-25, in the presence or absence of various bacterial stimuli. As shown in FIG. 2, IL-25 markedly inhibited the RNA expression of several inflammatory cytokines, i.e., IL-12, IL-23, IL-6, IL-8, and TNF-α induced by the presence of lipopolysaccharide (LPS) and peptidoglycan (PGN). CD14+ cells were less sensitive to stimulation by CpG motifs.

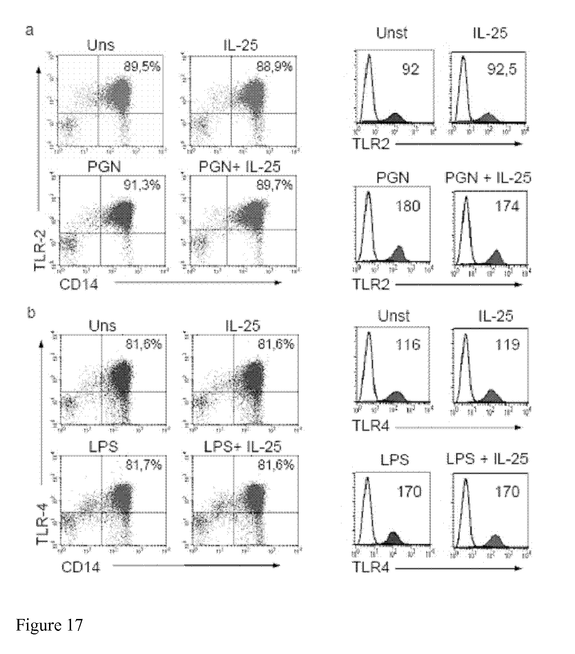

The induction of IL-23/p 19 and TNF-alpha by CpG was not modified by IL-25. One explanation for the IL-25-mediated reduced expression of LPS/PGN-stimulated inflammatory cytokines is the down-regulation of cell-surface receptors that mediate LPS/PGN signals. The expression of both toll-like receptor (TLR)-2 and TLR-4, at early (i.e., 6 hours, FIG. 17) and late (i.e., 48 hours, not shown) time points in cells either left unstimulated or stimulated with LPS or PGN was not modified by the presence of IL-25.

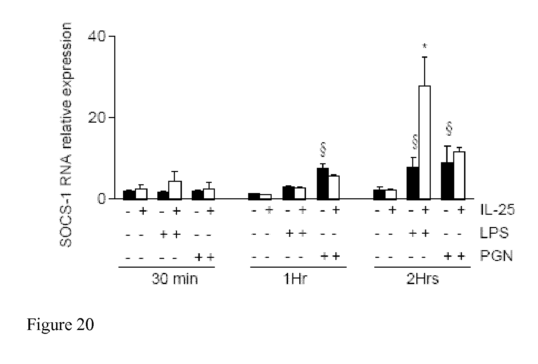

To assess whether IL-25 renders CD14+ cells resistant to additional inflammatory stimuli, CD14+ cells were pre-incubated with IL-25 for 30 minutes and then stimulated with IFN-gamma or TNF-alpha. As expected, the expression of IL-6 and IL-8 RNA transcripts was enhanced by TNF-alpha. Such an induction was significantly inhibited by IL-25 (FIG. 20A). Additionally, IL-25 reduced the expression of IL-6 and TNF-alpha transcripts induced by IFN-gamma (FIG. 20B).

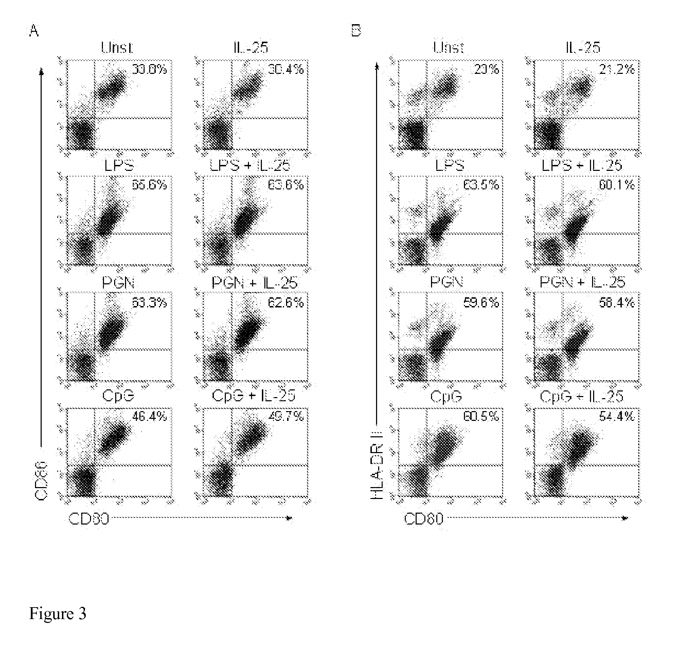

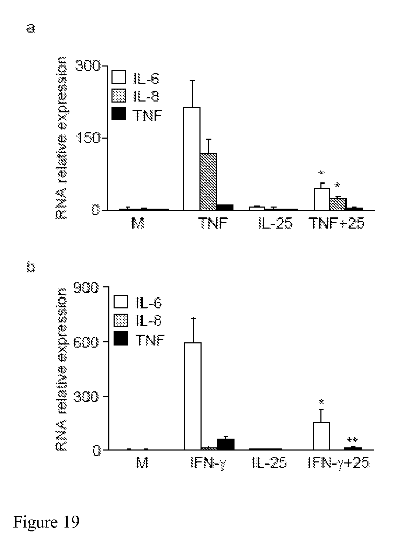

To exclude the possibility that IL-25 causes a global hyporesponsiveness of CD14+ cells, the expression levels of CD80, CD86, and HLA-DRII was investigated. CD14+ cells were treated with IL-25 or were left untreated. IL-25 did not reduce the fraction of CD80, CD86, and HLA-DRII induced by 6 and 48-hour stimulation with bacterial stimuli (FIG. 3 and not shown). Moreover, the ability of both unstimulated and LPS-stimulated CD14+ cells to phagocyte latex beads was not affected by IL-25 (FIG. 19). Overall these data indicate that IL-25 selectively inhibits the production of inflammatory cytokines rather than making CD 14+ cells resistant against bacterial/cytokine stimuli.

### Knockdown of SOC-3 by siRNA

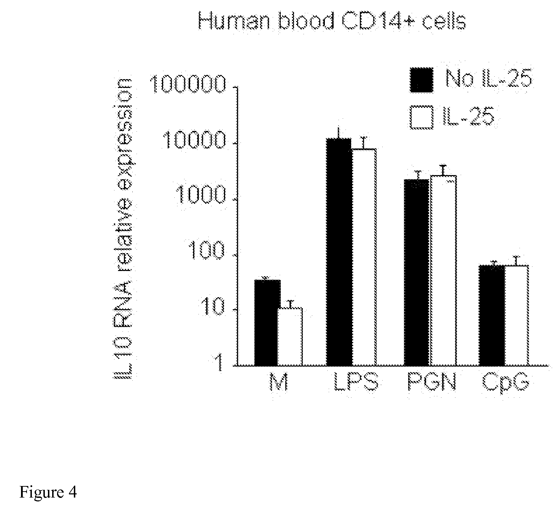

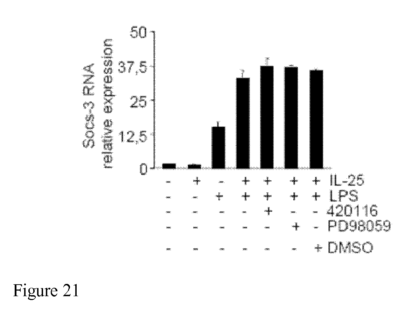

The effect of IL-25 on CD14+ cell response was examined for dependency on the induction and/ or activity of known negative regulators of monocyte/macrophage function. CD14+ cells were cultured with or without IL-25 in the presence or absence of LPS/PGN for different time points and then examined for expression of SOCS- and SOCS-3. LPS and PGN were effective in inducing SOCS-3. This effect was evident as early as 30 minutes after stimulation and was enhanced by IL-25 (FIG. 12A). LPS induced SOCS-1 RNA at 2 hours and this effect was enhanced further by pre-incubation of cells with IL-25 (FIG. 21). By contrast, induction of SOCS-1 RNA by PGN was seen at 1 hour, but it was not increased by IL-25 (FIG. 21). These results suggest that SOCS-3 but not SOCS-1 could be involved in the IL-25-mediated negative regulation of inflammatory cytokine by CD14+ cells. The expression of SOCS-3 in CD14+ cells was then eliminated by siRNA. CD14+ cells were stimulated with LPS or PGN in the presence or absence of IL-25. As shown in FIG. 12c, IL-25-mediated inhibition of both TNF-alpha and IL-6 expression following LPS or PGN stimulation was fully abrogated in SOCS-3-deficient cells. Induction of SOCS-3 in several cell types can be mediated by IL-10, an antiinflammatory cytokine that negatively regulates production of inflammatory cytokines by CD14+ cells. Therefore, the expression of IL-10 RNA was analyzed in cells cultured with or without IL-25 in the presence or absence of bacterial stimuli. However, no significant change in the expression of IL-10 was seen in IL-25-treated cells (FIG. 4). Overall these results indicate that IL-25 is a negative regulator of monocyte/macrophage inflammatory cytokine synthesis.

### IL-25R expression in Inflammatory Bowel Disease (IBD)

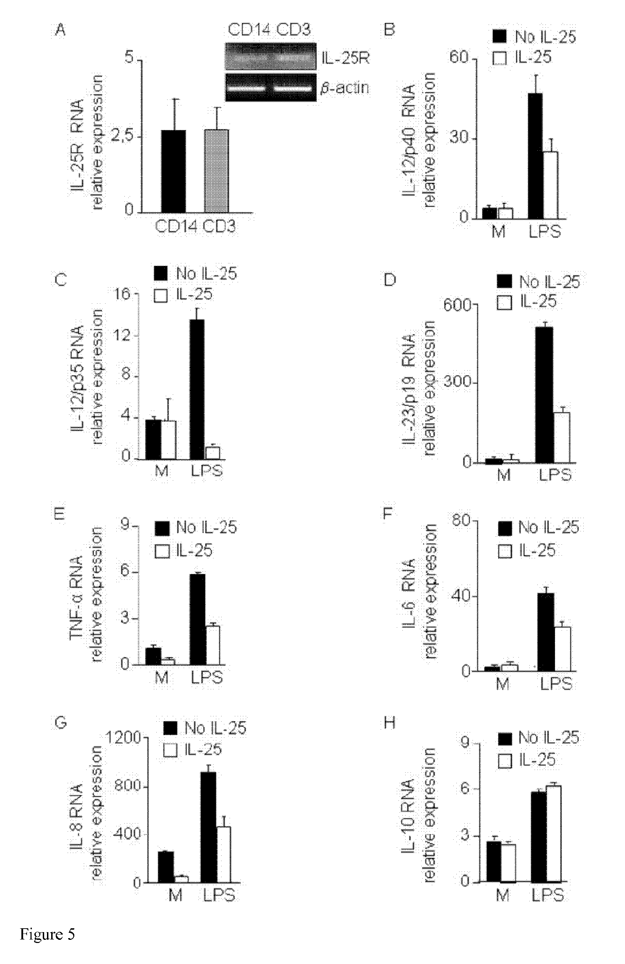

The expression of IL-25R in CD 14+ cells isolated from the colon of patients with IBD was analyzed to investigate if these CD 14+ cells are functionally capable of responding to IL-25. Real-time PCR analyses showed that IL-25R transcripts were present in mucosal Crohns disease (CD) CD14+ cells (FIG. 5A). These cells were also shown to express the soluble IL-25R isoform (FIG. 5A, right inset). The treatment of CD CD14+ cells with IL-25 resulted in a significant reduction in the RNA content of inflammatory cytokines induced by LPS (FIG. 5B-G). IL-25 also reduced the constitutive RNA expression of TNF-alpha (FIG. 5E) and IL-8 (FIG. SG). In line with data obtained in peripheral blood CD14+ cells, IL-25 did not modify the LPS-induced IL-10 RNA content (FIG. 5H).

### Expression of IL-25 in colon.

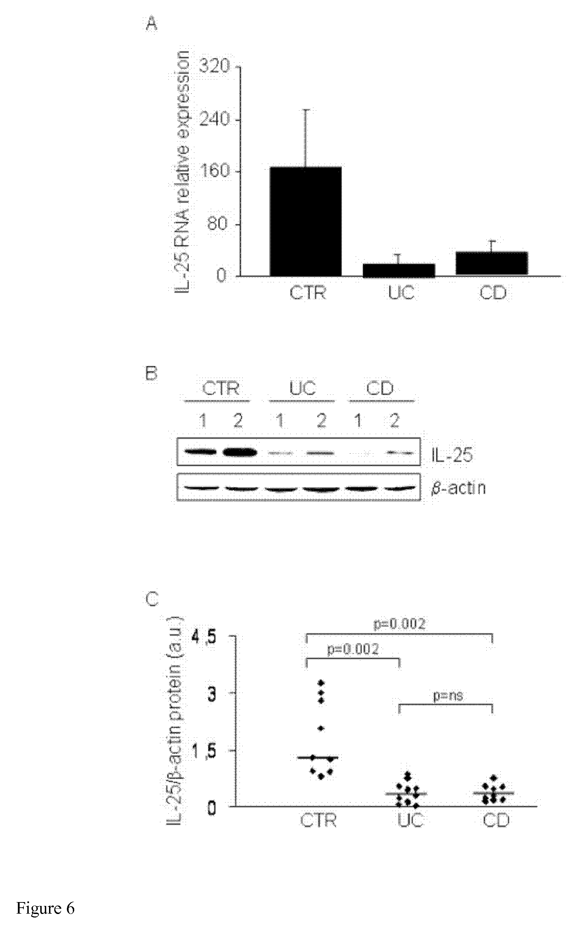

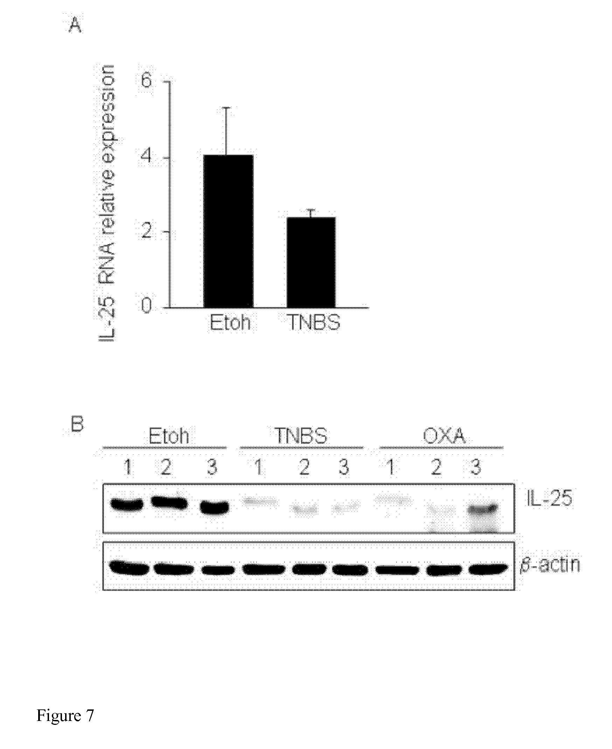

The expression of IL-25 in the human colon was investigated. A constitutive expression of IL-25 RNA and protein was seen in normal colonic samples. Surprisingly, however, a marked down-regulation of IL-25 was seen in tissues from patients with IBD in comparison to the controls, and this was evident at both the RNA (FIG. 6A) and protein level (FIG. 6 B-C). Consistently, IL-25 RNA expression was down-regulated in mice with trinitrobenzene sulfonic acid (TNBS)-colitis, that shows similarities with CD, as compared with ethanol-treated controls (FIG. 7A). Moreover, Western blotting analysis of IL-25 protein content in colonic extracts showed that IL-25 expression is markedly reduced in mice with TNBS or oxazolone-colitis in comparison to controls (FIG. 7B).

### PGN-induced colitis in a mouse model

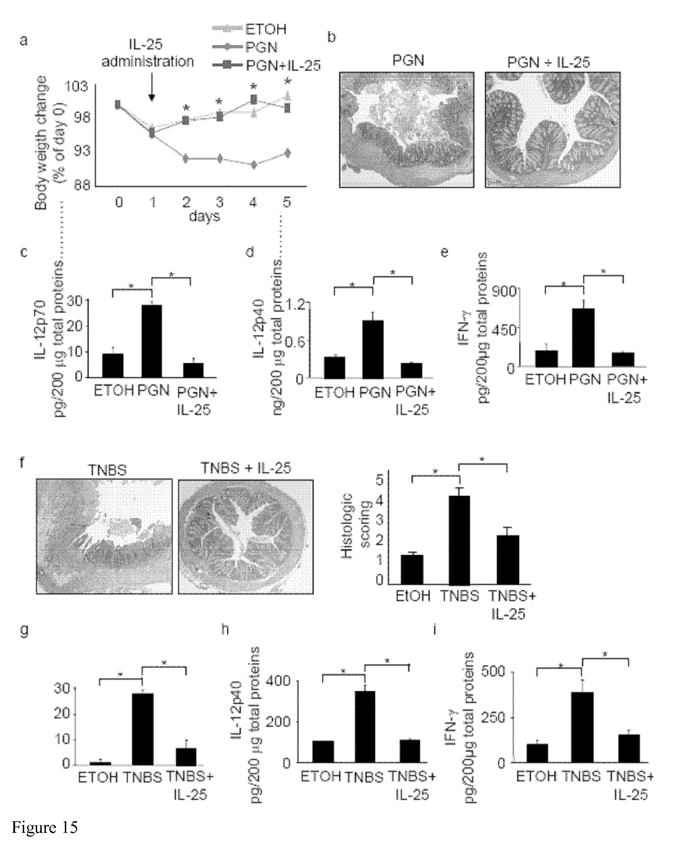

A mouse model of PGN-induced colitis was investigated for response to intraperitoneal injection of IL-25. The colitis was inducible by rectal administration of PGN. As shown in FIG. 15(a), intraperitoneal injection of IL-25 prevented the weight loss induced by PGN. Severe mucosal mononuclear cell infiltrate and distortion of the normal crypt architecture with epithelial ulceration and loss of goblet cells was evident in the colon of mice with PGN receiving no IL-25. In contrast, only small scattered areas of cellular infiltration is seen in the colon of mice treated with IL-25 (FIG.15 (b)). Cytokine levels in tissue samples were analyzed by ELISA and data expressed as pg/200 µg total proteins. Each point on the graph illustrated in FIG. 15 (c) represents the value of cytokine in colonic samples taken from a single mouse. The horizontal bars indicate the median value.

### IL-25 ameliorates induced PGN-mediated colitis

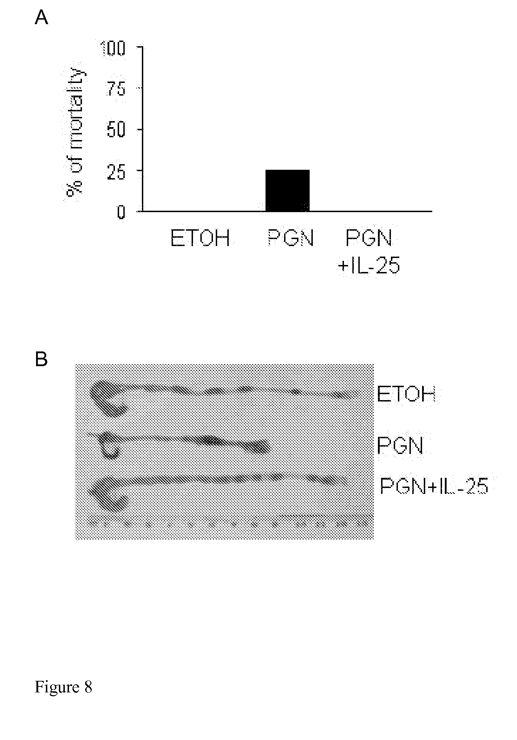

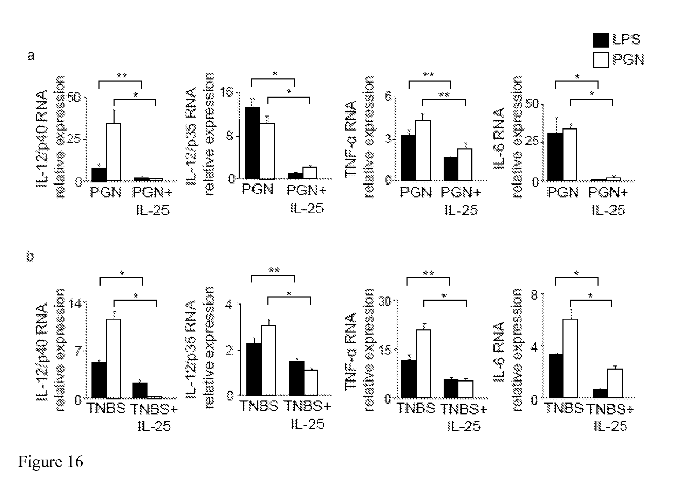

PGN-colitis was induced in Balb/c mice. Control mice were given intrarectal administration of 50% ETOH no PGN. Recombinant mouse IL-25 was administered the day after the induction of colitis. FIG. 8 (a) shows a 25% rate of mortality in mice suffering from PGN-mediated colitis left untreated. A 0% rate in mortality was observed in mice suffering from PGN-mediated colitis treated with IL-25 and control mice. IL-25 administration on day 1 resulted in an increase in body weight in mice with PGN-mediated colitis compared to those mice left untreated (FIG. 16 (a)). Furthermore, on day 5 there was a significant decrease in body weight in mice suffering with PGN-mediated colitis who were left untreated compared to control mice and those treated with IL-25.

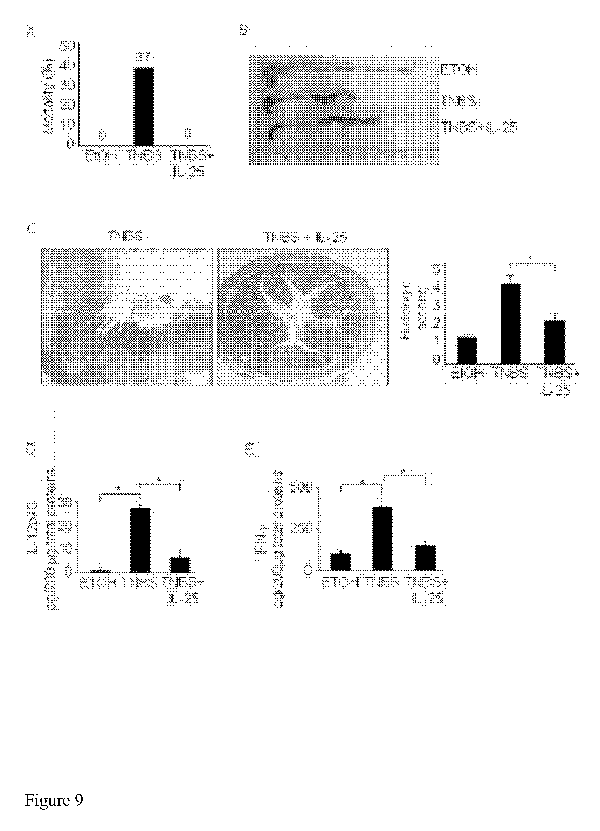

IL-25 was administered to mice with TNBS-induced colitis the day after induction. Mice exhibited a loss of body weight independently of IL-25 treatment (data not shown). A mortality rate of 37% was observed in mice untreated with IL-25. A 0% mortality rate was seen in mice treated with IL-25. Macroscopically, colon samples obtained from mice left untreated appeared shorter than those obtained from mice receiving treatment, as displayed in FIG. 9 (b). Consistently, blinded histological scoring of colon tissue was significantly reduced in mice treated with IL-25 compared to mice left untreated (FIG. 9 (c)). The effect of IL-25 on Th-1 cytokine production in mice with TNBS-induced colitis was examined. The production of both heterodimeric IL-12/p70 and IFN-γ was significantly increased in the colons of mice with TNBS-colitis in comparison to ethanol treated controls (FIGs. 9 (d)(e)). Subsequent, administration of IL-25 to mice with TNBS-colitis significantly reduced the production of both of these cytokines.

### Discussion

CD14+ cells play a central role in regulation of cytokine production observed during the progression of inflammatory diseases such as Inflammatory Bowel Disease (IBD). It is well known in the art that CD14+ cells are present in the intestinal mucosa and produce inflammatory cytokines, IL-1beta, IL-6, TNF-alpha and IL-8. The molecular mechanisms underlying the enhanced production of inflammatory cytokines by CD 14+ cells in IBD are not, however, fully understood. Presently employed methods of treatment for Inflammatory Bowel Disease are plagued with the occurrence of severe side effects and resistance among patients. The present invention provides for an alternative medicament for use in the treatment of Inflammatory Bowel Disease. The present invention relates to a means to regulate the cytokine response of CD 14+ cells.

Interleukin (IL)-25 is a cytokine thought to be involved in the inflammatory response. However, the precise role IL-25 plays in such a response is presently undefined. The current inventors have shown that the receptor responsible for mediating the biological functions of IL-25, namely IL-25R, is present on the surface of CD14+ cells in humans suggesting that CD 14+ cells are potential targets of IL-25. The current inventors are the first to show that there is, surprisingly, a defective amount of IL-25 in the inflamed colon of patients with IBD and in experimental mouse models of IBD in comparison to normal control samples.

The current inventors have further shown that IL-25 is a negative regulator of the cytokine response by CD14+ cells in the presence of bacterial and inflammatory stimuli. In addition, IL-25 did not reduce the expression of CD80, CD86 and HLA-DRII, induced by bacterial stimuli in CD 14+ cells isolated from the colon of patients with IBD, indicating the IL-25 does not cause a global hypo-responsiveness but rather selectively inhibits the production of inflammatory cytokines. These findings indicate a stimulus-independent inability of CD14+ cells to produce inflammatory cytokines following exposure to IL-25.

The treatment of CD14+ cells isolated from patients suffering from IBD, with IL-25, resulted in a significant reduction in the RNA content of inflammatory cytokines induced by LPS. IL-25 also reduced the constitutive RNA expression of TNF-alpha and IL-8. The current inventors induced PGN-mediated colitis in a mouse model in order to examine the effect of IL-25 administration. IL-25 administration in mice prevented the induction of PGN-mediated colitis. Histological evaluation of colon samples from mice treated with IL-25, revealed only small, scattered areas of cellular infiltration following administration of PGN. Furthermore, a significant improvement was observed in mice suffering from PGN-mediated colitis following treatment with IL-25 compared to those mice with PGN-mediated colitis left untreated. An additional mouse model of colitis, TNBS-induced colitis, was utilized to evaluate and further confirm the potential therapeutic properties of IL-25. Administration of IL-25 to mice suffering from TNBS-colitis led to a 0% rate of mortality among mice compared to a 37% rate of mortality among mice left untreated. IL-25 treatment also significantly reduced the production of the Th-1 cytokine response in the colon of mice.

The effect of IL-25 on CD14+ cell response was examined for dependency on the induction and/or activity of known negative regulators of monocyte/macrophage function. These results suggest that SOCS-3 but not SOCS-1 could be involved in the IL-25-mediated negative regulation of inflammatory cytokine by CD 14+ cells.

Taken together, the data suggests that a defective IL-25-mediated down-regulation of cytokine response can contribute to amplify monocyte/macrophage cell-mediated immune-inflammatory pathologies. These results indicate that administration of IL-25 could be a potential method of treatment for diseases in which CD14+ plays a part such as Inflammatory Bowel Disease.

### Materials and Methods

### Cell Culture

Human peripheral blood mononuclear cells (PBMC) were isolated from enriched buffy coats of healthy volunteer donors and patients with IBD. PBMC were used as a source for the isolation and purification of CD14+ and CD3+ T cells, carried out using CD14 or CD3 magnetic beads (Miltenyi Biotec, Bologna, Italy). Cell purity was routinely evaluated by flow cytometry and ranged between 96% and 98%. All reagents were from Sigma-Aldrich (Milan, Italy) unless specified. RNA was extracted from an aliquot of PBMCs and assessed for IL-25R expression by flow-cytometry. The remaining cells were resuspended in RPMI 1640 medium, supplemented with 10% inactivated fetal bovine serum (FBS), penicillin (100 U/mL), and streptomycin (100 µg/mL) (Life Technologies-GibcoCRL, Milan, Italy), and seeded in 48-well culture dishes (1x106 cells/well). CD14+ cells were pre-incubated with IL-25 (50 ng/ml, R&D Systems, Inc. Minneapolis, MN, USA) for 30 minutes and then stimulated with LPS (100 ng/ml) or PGN (10µg/ml) or CpG (5µg/ml) or TNF-α (R&D Systems), or IFN-γ (50 ng/ml, Peprotech EC LTD, London, UK) for 30 minutes to 48 hours. RNA was then extracted from the cells for cell surface protein analysis.

### RNA extraction and cDNA preparation

RNA was extracted from TRIzol reagent according to the manufacturer's instructions (Invitrogen, Milan, Italy). A constant amount of RNA (500 ng/sample) was retrotranscribed into complementary DNA (cDNA).

### Real-time PCR

1 µl of cDNA from each sample was amplified using the following conditions outlined in Table 1.

**Table 1 Real-Time PCR conditions**

| **Step** | **Time** | **Temperature** |
|---|---|---|
| Denaturation | 1 min | 95°C |
| Annealing | 30 sec | 58°C (human IL-23/p19, human IL-8) |
| | | 60°C (human IL-6) |
| | | 62°C (human TNF-α, human IL-17A, human IL-25R membrane-bound isoform, murine/β-actin) |
| Extension | 30 see | 72°C |

Primers sequences used in the current invention are as listed in Table 2

**Table 2: Primers sequences**

| **Primer Name** | **Primer Sequence** |
|---|---|
| Human IL-23/p19 FWD | 5'-GGGACACATGGATCTAAGAG-3' |
| Human IL-23/p19 REV | 5'-GCAAGCAGAACTGACTGTTG-3' |
| Human IL-8 FWD | 5'-AGGAACCATCTCACTGTGTG-3' |
| Human IL-8 REV | 5'-CCACTCTCAATCACTCTCAG-3' |
| Human IL-6 FWD | 5'-CCACTCACCTCTTCAGAACG-3' |
| Human IL-6 REV | 5'-GCCTCTTTGCTGCTTTCACAC-3' |
| Human TNF-α FWD | 5'-AGGCGGTGCTTGTTCCTCAG-3' |
| Human TNF-α REV | 5'-GGCTACAGGCTTGTCACTCG-3' |
| Human IL-17A FWD | 5'-ACTACAACCGATCCACCTCAC-3' |
| Human IL-17A REV | 5'-ACTTTGCCTCCCAGATCACAG-3' |
| Human IL-25R membrane-bound isoform FWD | 5'-CCTCCGAGTAGAACCTGTTAC-3' |
| Human IL-25R membrane-bound isoform REV | 5'-AGTTGCTTTTGCCCGTCACAC-3', |
| Human IL-25 soluble form FWD | 5'-AGGTGGGGACACAGGAGGTC-3' |
| Human IL-25 soluble form REV | 5'-CTGGAGGACACAGGGGTGGA-3'. |
| β-actin FWD | 5'AAGATGACCCAGATCATGTTTGAGACC-3' |
| β-actin REV | 5'-AGCCAGTCCAGACGCAGGAT-3 |

The expression levels of murine and human IL-25, human IL-23/p40, human IL-12/p35, human SOCS-1 and SOCS-3 were evaluated using a commercially available TaqMan probe (Applied Biosystems, Foster City, CA, USA). β-action was used as an internal control. Real-time PCR was performed using the IQ SYBR Green Supermix (Bio-Rad Laboratories, Milan, Italy).

### Flow-Cytometry

The level of IL-25R was evaluated in total PBMC and in purified blood and intestinal CD3+ T and CD14+ cells using the following monoclonal anti-human antibodies:
❖ CD56 FITC (1:10 final dilution, Diaclone Research, Milan, Italy)
❖ CD3 PerCP (1:50, final dilution, Becton Dickinson, Milan, Italy)
❖ CDR14 FITC (1:50 final dilution, Immunotools, Friesoythe; Germany)
❖ CD19 (1:50 final dilution, Immunotools)
❖ Isotype control IgG (Becton Dickinson, Milan, Italy)
❖ IL-25R PE (1:10 final dilution, R&D Systems)

To evaluate cell activation markers, CD 14+ cells were targeted by the following antibodies:
❖ anti-human TLR4 PE or TLR2 PE (both at 1:5 final dilution, eBioscience, San Diego, CA, USA)
❖ anti-human HLD-DRII PE or anti-human CD86 FITC (both at 1:50 final dilution, Becton Dickinson)
❖ anti-human CD80 APC (1:50 final dilution, Immunotools)

Cells were then incubated at 4°C for 30 minutes. Cells were washed, resuspended in PBS and analyzed by flow-cytometry. In order to determine the effect of IL-25 on the phagocytic properties of CD14+ cells, CD14+ cells were seeded in a 6-well dishes (1x10⁶ cells/ml) and pre-incubated with IL-25 (50 ng/ml) for 30 minutes and then stimulated with LPS (100 ng/ml) or PGN (10µg/ml) in presence of orange fluorescent latex beads (5x10⁶ beads/ml) for 6 hours at 37°C. Cells were then washed, resuspended in PBS and analyzed by flow-cytometry.

### SOCS-3

To examine the role of SOCS-3 in the IL-25-mediated negative regulation of CD14+ cell cytokine response, CD14+ cells were cultured with and without human SOCS-3 or control siRNA according to the manufacturer instructions (Santa Cruz Biotechnology, Santa Cruz, CA). After 48 hours, cells were washed and cultured with or without IL-25 for 30 minutes and then stimulated with LPS or PGN for further 6 hours.

### Tissue Samples

Colonic biopsies were taken during endoscopy from inflamed areas of 9 patients with CD. In 5 out of 9 CD patients, the primary site of involvement was the terminal ileum and colon, while the remaining patients had a colonic disease. Two CD patients were receiving treatment with mesalazine, 5 patients were receiving steroids and mesalazine and the remaining patients were taking antibiotics. Additional mucosal samples were taken from intestinal resection specimens of 4 patients undergoing surgery for a chronic active disease poorly responsive to medical treatment. At the time of surgery, 2 patients were receiving corticosteroids and 2 patients were receiving antibiotics and corticosteroids.

Control samples include 12 patients with ulcerative colitis (UC): 6 with a left-sided colitis and 6 with an extensive disease. Two UC patients were on oral and topical mesalazine and the remaining patients were receiving steroids and topical mesalazine. "Normal" control samples include colonic mucosal samples taken from macroscopically and microscopically unaffected areas of 9 subjects undergoing colonoscopy for colorectal cancer screening, and 8 subjects undergoing colon resection for colon cancer.

### Lamina propria mononuclear cell isolation and cell culture

Lamina propria mononuclear cells (LPMC) were prepared by using the DTT-EDTA-collagenase procedure and used as a source for the purification CD14+ and CD3+ T-cells by positive MACS separation. The resulting cell preparations contained >95% CD14+ and >95% CD3+ T cells, as assessed by flow cytometry. RNA was extracted from an aliquot of cells and assessed for the expression of IL-25R by real-time PCR and flow cytometry. The remaining cells were resuspended in RPMI 1640 medium, supplemented with 10% inactivated FBS, penicillin (100 U/mL), and streptomycin (100 ng/mL), and seeded in 48-well culture dishes (1x106 cells/well). CD14+ cells were cultured with and without IL-25 (50 ng/ml) for 30 minutes and then stimulated with LPS. CD3+ T cells were cultured with or without IL-25 (50 ng/ml) for 30 minutes and then stimulated with activating anti-CD3+anti-CD28 antibodies. After 6 hours, cells were harvested and used for RNA extraction.

### Induction of trinitrobenzene sulfonic acid (TNBS) and Oxazolone colitis

Studies of hapten-induced colitis were performed in 5- to 6-week-old male SJL mice (Harlan Laboratories, S. Pietro al Natisone, UD, Italy), which were maintained in the animal facility at the Istituto Superiore di Sanità (Rome, Italy). The experiments were performed within 7 days of the arrival of the animals. For induction of colitis, 2.5 mg of TNBS or 6 mg of oxazolone in 50% ethanol was administered to lightly anesthetized mice through a 3.5F catheter inserted into the rectum. The catheter tip was inserted 4 cm proximal to the anal verge, and 150 µl of fluid was slowly instilled into the colon, after which the mouse was held in a vertical position for 30 seconds. Controls consisted of mice treated with 150 µL of 50% ethanol and untreated naive mice. Weight changes were recorded daily to assess the induction of colitis, and tissues were collected for histologic study and protein analysis. For histologic analysis, tissues were fixed in 10% neutral buffered formalin solution, embedded in paraffin, cut into tissue sections, and stained with H&E. For TNBS-induced colitis, stained sections were examined for evidence of colitis and assigned a colitis score by considering the presence of acute and chronic inflammatory infiltrates, elongation and/or distortion of crypts, frank ulceration, and thickening of the bowel wall. Additionally, colonic tissues taken from TNBS-induced colitis was used for RNA and protein extraction.

For oxazolone-induced colitis, stained sections were examined and assigned a colitis score by examining the slide for the presence of hypervascularization, mononuclear cells, epithelial hyperplasia, epithelial injury, and granulocytes. Colonic tissues taken from Oxazolone-induced colitis were used for protein extraction.

### Induction of Peptidoglycan (PGN) colitis and IL-25 administration

PGN colitis was induced in Balb/c mice by intrarectal administration of 100 µl of 50% ethanol followed 8 hours later by soluble PGN (1mg/mouse) again per rectum. Controls included mice that were given intrarectal administration with 50% ethanol but not PGN. Recombinant mouse IL-25 (10µg/mouse) was injected intra-peritoneally 1 hour before the administration of PGN, in the preventive model, whereas the cytokines was injected 1 day after the administration of PGN, in the therapeutic model. Weight changes were recorded daily to assess the induction of colitis, and tissues were collected for histologic study, protein analysis, and isolation of splenocytes and colonic LPMC. For histologic analysis, tissues were fixed in 10% neutral buffered formalin solution, embedded in paraffin, cut into tissue sections, and stained with H&E. In order to evaluate the effect of treatment with IL-25 in PGN-mediated colitis, IL-25 was administered (10µg/mouse) to Balb/c mice the day after the induction of colitis. Body weight changes were recorded daily in control and colitic mice either treated or untreated with IL-25.

### Protein extraction, Western blotting and IL-12 ELISA

IL-25 protein was analyzed using total proteins extracted from biopsies of 8 CD patients, 8 UC patients and 8 normal controls. Additionally, IL-25 was evaluated in colonic samples of 6 TNBS-induced colitis mice, 3 Oxazolone-induced colitis mice and 6 controls. Samples were lysed for 60 min on ice in buffer containing 10 mM Hepes (pH 7.9), 10 mM KC1, 0.1 mM EDTA, 0.2 mM EGTA, and 0,5% Nonidet P40, supplemented with 1 mM DTT, 10 µg/ml aprotinin, 10µ g/ml leupeptin, 1 mM phenylmethanesulphonyl fluoride (PMSF), 1 mM Na3V04, and 1 mM NaF (all reagents were from Sigma-Aldrich). Lysates were then clarified by centrifugation at 4 °C for 30 minutes at 12,000 x g. Equal amounts of total proteins were fractionated on SDS-polyacrylamide gels. The membranes were blocked with Tris-buffered saline containing 0.05% Tween 20 and 5% nonfat dry milk and then incubated with a mouse anti-human IL-25 (1 µg/ml) or with a rat antimouse IL-25 (1 µg/ml, both from R&D Systems) followed by a horseradish peroxidase-conjugated secondary antibodies (1:20000 final dilution, Dako, Milan, Italy). The reaction was detected with a chemiluminescence kit (West DURA; Pierce, Rockford, IL). Computer-assisted scanning densitometry (Total lab, AB.EL S.r.1., Rome, Italy) was used to analyze the intensity of the immunoreactive bands.
Total proteins extracted from mice with PGN-colitis and controls either left untreated or treated with IL-25 were used to analyze IL-12/p70 by a commercially available ELISA kit (R&D Systems) according to the manufacturer's instructions

### EXAMPLE 2

IL-25 (also known as IL-17E) is a recently described member of the IL-17 cytokine gene family. IL-25 is made by several cell types, including T lymphocytes, mast cells activated by IgE cross-linking (Ikeda et al., 2003), alveolar macrophage after particle inhalation (Kang et al., 2005), and activated eosinophils and basophils (Wang et al., 2007). IL-25 can also be produced by lung epithelial cells and alveolar macrophages upon allergen stimulation (Angkasekwinai et al., 2007). IL-25 biological activity is mediated by a transmembrane receptor (IL-25R), also called IL-17BR or IL-17R homolog1, that is constitutively expressed in the liver, kidneys, and intestine (9, 10) **A LIST OF THE "REFERENCES" WAS NOT PROVIDED IN THE MANUSCRIPT DOCUMENT, BUT CITATIONS (E.G., (9, 10)) ARE MADE IN THE TEXT???.** Distinct from other IL-17 cytokine family members, IL-25 has been shown to facilitate pathogenic Th2 cell responses. Indeed, studies in mice have shown that both transgenic expression of IL-25 and systemic administration of recombinant cytokine increase the production of IL-4, IL-5, and IL-13, cause epithelial cell hyperplasia, and facilitate the recruitment of inflammatory cells (i.e., eosinophils, monocytes, and T cells) into inflamed tissues (9, 11, 12). IL-25 also sustains Th2 cytokine production and airway inflammation in an allergen-induced asthma model. The ability of IL-25 to promote Th2 cell response is substantiated further by studies in murine models of helminth parasite infection. IL-25-knockout mice fail to expel the parasitic helminthes Nippostrongylus brasiliensis and Trichuris muris from the gut, a defect that correlates with their inability to up-regulate Th2 cytokines (13, 14). Consistently, administration of exogenous IL-25 to genetically susceptible mice confers resistance to helminth infection and accelerates expulsion of worms (14).

More recently, IL-25 has been reported to control the activity of other cell types. For example, studies in murine models of autoimmunity have shown that IL-25 can negatively regulate the development and/or amplification of Th17-mediated pathology in a strictly IL-13-dependent fashion (15). Altogether these observations raise the possibility that IL-25 may both trigger and abrogate specific inflammatory responses. However, the cell types that respond to IL-25 and the mechanisms by which IL-25 can differently regulate immune reactions are not entirely known.

Here we show that human blood and intestinal mucosal CD14+ cells express high levels of IL-25R, and respond to IL-25 by down-regulating the expression of inflammatory cytokines induced by toll-like receptor (TLR) ligands (i.e., LPS, PGN) or inflammatory cytokines [i.e., tumor necrosis factor (TNF)-α and interferon (IFN)-γ]. Inhibition of cytokine response by IL-25 occurs via a p38 MAP kinase (MAPK)-driven suppressor of cytokine signaling (SOCS)-3 induction-dependent mechanism. Consistently, IL-25 provides protection from LPS-induced lethal endotoxemia, and largely prevents and reverses experimental colitis. Overall these data indicate that IL-25 negatively regulates monocyte-derived cytokines, thus suggesting that IL-25 therapy may be useful for attenuating monocyte-associated immune diseases.

### RESULTS

### IL-25R is highly expressed by human CD14+ cells

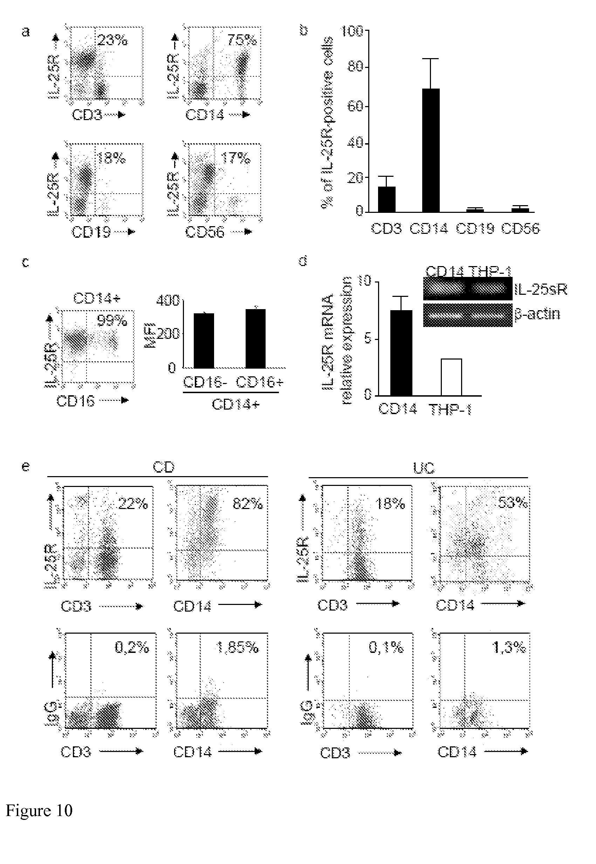

To begin to understand which cells are potential targets of IL-25, we assessed the expression of IL-25R in human peripheral blood mononuclear cells (PBMC) by flow-cytometry. As shown in the representative dot-plot experiment in figure 10(a), 75% of CD14+ cells expressed IL-25R. IL-25R was also detected in less than 25% of CD3+, CD19+, and CD56+ cells (FIG. 10(a)). To exclude that our data were somewhat influenced by the density gradient separation, we analyzed IL-25 in fresh blood samples, and showed that it was preferentially expressed by CD14+ cells (FIG. 10(b)). Recent studies have identified a subset of blood CD 14+ cells that expresses high levels of CD 16, the Fc receptor II, and is capable of producing huge amounts of inflammatory cytokines. So, we determined whether IL-25R is expressed by CD14+CD16+ cells. To this end, blood CD14+ cells were simultaneously evaluated for IL-25R and CD16. The representative dot-plot shown in FIG. 10(c) indicates that nearly all CD14+CD16+ cells expressed IL-25. Moreover, there was no apparent difference in the mean fluorescence intensity of IL-25 expression between CD16- cells and CD16+ cells (FIG. 10(c) right inset). To confirm further our results, we showed that human blood CD14+ cells and The-1, a human monocytic cell line, contain transcripts for IL-25R (FIG. 10(d)). Moreover, both human blood CD14+ cells and THP-1 cells express transcripts for the soluble IL-25R isoform, whose function remains however to be ascertained (FIG. 10(d), right inset).

The gastrointestinal tract mucosa contains the largest reservoir of macrophages in the body. In the normal gut, macrophages do not express CD14 and display markedly reduced LPS-induced inflammatory responses. However, during inflammatory processes, such as Crohn's disease (CD) and ulcerative colitis (UC), the two major forms of inflammatory bowel diseases (IBD) in humans, CD14+ blood monocytes are recruited to the mucosa where, presumably in response to local stimulatory signals, they produce huge amounts of inflammatory mediators that promote tissue inflammation and damage. Therefore, we next analyzed IL-25R expression in lamina propria mononuclear cells (LPMC) isolated from the inflamed colon of IBD patients. In line with the above results, more than 50% of CD14+ cells and approximately 20% of CD3+ T cells isolated from both CD and UC tissues expressed IL-25R (FIG. 10(e)). Overall these data indicate that IL-25 is expressed by blood and mucosal CD14+ cells and suggest that CD14+ cells are potential targets of IL-25.

### IL-25 inhibits the cytokine response of CD14+ cells following stimulation with TLR ligands and inflammatory cytokines

CD14+ cells express high levels of toll-like receptors (TLRs) and are capable of producing huge amounts of cytokines in response to TLR ligands. Therefore, to gain new insights into the function of IL-25, human blood CD14+ cells were pre-incubated with IL-25 for 30 min and then exposed to TLR ligands for further 6 hours. At the end, the expression of a large panel of cytokines was examined by real-time PCR. As shown in Table 1, IL-25 pretreatment has a marked inhibitory effect on the expression of inflammatory cytokines induced by PGN and LPS. By contrast, IL-25 did not modify the expression ofIL-10 induced by both LPS and PGN. Data were confirmed by showing that secretion ofIL12p40 and TNF-α induced by 48-hour stimulation with LPS (518 ± 110 and 310 ± 42 pg/ml respectively) or PGN (118 ± 49 and 220 ± 55 pg/ml respectively) was significantly reduced by pre-incubation of cells with IL-25 (167 ± 103 and 91 ± 31 pg/ml for IL-12p40, and 95 ± 26 and 60 ± 12 pg/ml for TNF-α, p<0.02).

PGN and LPS signal through TLR-2 and TLR-4 respectively. So, we next examined whether the ability of IL-25 to antagonize the LPS/PGN response was due to changes in the expression of these two receptors. Treatment of CD14+ cells with IL-25 did affect neither the percentage of TLR-2 and TLR-4-positive cells nor the average median fluorescence intensity of TLR expression, and this was evident at early (i.e., 6 hours, FIG. 17) and late (i.e., 48 hours, not shown) time points. It is also unlikely that the above inhibition of TLR2 and TLR4 responses by IL-25 pretreatment was due to a toxic effect of the cytokine, as no significant change in the percentages of Annexin V (AV) and/or propidium iodide (PI)-positive cells was seen after IL-25 stimulation (not shown). Additionally, IL-25 did alter neither the expression of CD80, CD86, and HLA-DRII induced by

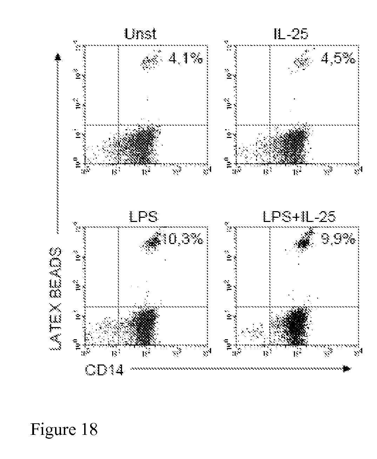

LPS/PGN (FIG. 3) nor the ability of CD14+ cells to phagocyte fluorescent beads after LPS stimulation (FIG. 18).

We also evaluated the effect of IL-25 on RNA expression of inflammatory cytokines in mucosal CD14+ cells isolated from patients with IBD. In these studies, cells were pre-incubated with IL-25 for 30 min and then stimulated with LPS for additional 6 hours. IL-25 pre-stimulation led to a marked down-regulation of LPS-induced cytokine expression (Table 2). By contrast, IL-25 did not significantly affect the LPS-induced IL-10 expression (Table 2).

Treatment of blood CD14+ cells with IL-25 also reduced the RNA expression of IL-6, IL-8, and TNF-α following stimulation with TNF-α and IFN-γ (FIG. 19), thus indicating that IL-25 makes CD14+ cells resistant to multiple inflammatory signals.

### Inhibition of CD14+ cell cytokine response by IL-25 requires SOCS-3

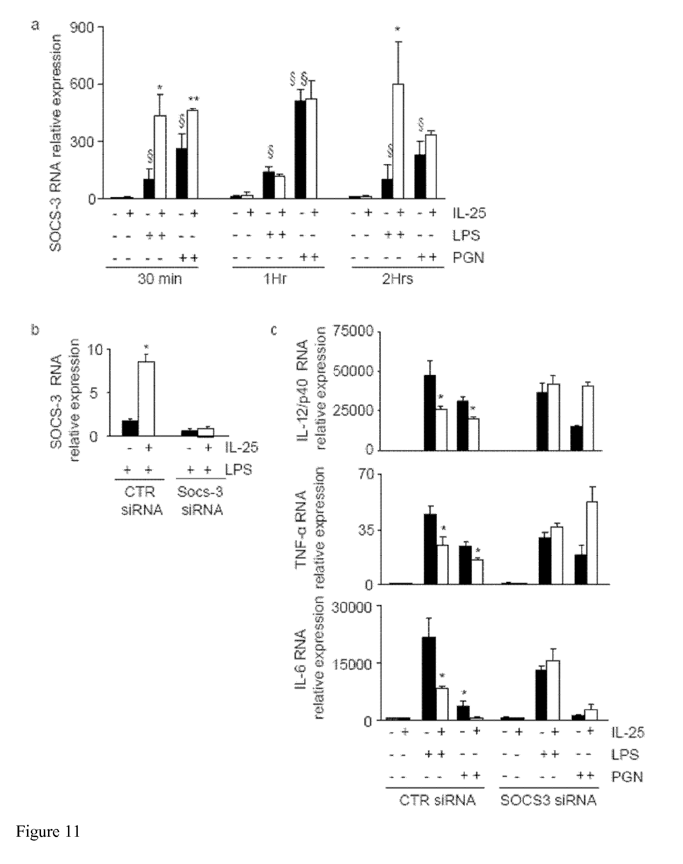

SOCS-1 and SOCS-3 are negative regulators of both TLR and cytokine-induced JAK-Stat signaling pathways. Therefore, we determined whether SOCS1 and/or SOCS-3 play a role in the IL-25-mediated negative regulation of cytokine response in CD14+ cells. To address this issue, we initially evaluated the RNA content of these molecules in CD 14+ cells stimulated with IL-25 in the presence or absence of LPS/PGN. SOCS-1 RNA expression was induced by LPS at 2 h and this effect was significantly enhanced by IL-25 (FIG. 20). SOCS-1 RNA expression was also enhanced by PGN at 1 and 2 h, but no further increase was seen in cells stimulated with PGN and IL-25 (FIG. 20). Analysis at later time points confirmed these results (not shown). Stimulation of CD14+ cells with LPS significantly increased SOCS-3 RNA expression at each time point, and this effect was markedly augmented by IL-25 at 0.5 and 2 h (FIG. 11(a)). SOCS-3 RNA expression was also induced at 0.5 h after the addition of PGN, peaked at 1 h, and diminished at 2 h. Notably, the combination of IL-25 and PGN exerted a greater effect on the induction of SOCS-3 RNA at 0.5 h than that seen in cells stimulated with PGN alone (FIG. 11(a)). Based upon these data, we thought that SOCS-3 rather than SOCS-1 could be involved in the IL-25-negative regulation of CD14+ cell response to LPS and PGN. We thus assessed whether gene silencing of SOCS-3 expression by siRNA abolished the IL-25-mediated inhibitory effects on CD 14+ cell cytokine response. To this end, CD14+ cells were transfected with a human SOCS-3 or control siRNA. After 48 h, cells were extensively washed, cultured in the presence or absence of IL-25 for 30 minutes and then stimulated with LPS or PGN for further 2-6 hours. At the end, RNA expression of SOCS-3, IL-12/p40, TNF-α, and IL-6 was evaluated by real-time PCR. As expected, treatment of cells with SOCS-3 siRNA led to a marked inhibition of SOCS-3 (FIG. 11(b)) but not SOCS-1 (not shown) RNA expression. Importantly, the expression of IL-12/p40, TNF-α, and IL-6 induced by LPS and PGN was not down-regulated by IL-25 in SOCS-3-deficient CD14+ cells (FIG. 11(c)), thus supporting the role of SOCS-3 in the IL-25-mediated inhibition of CD 14+ cell cytokine response.

### Induction of SOCS-3 by IL-25 is dependent on p38 MAP kinase activity

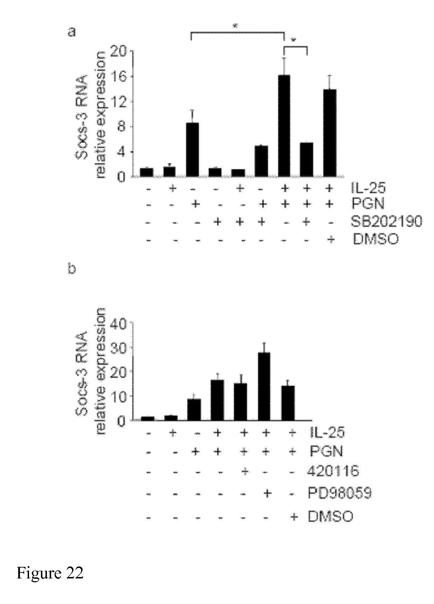

SOCS-3 gene is transcriptionally regulated by signal transducers and activators of transcription (Stat), mostly Stat3. However, treatment of CD14+ cells with IL-25 did not enhance Stat3 activation (not shown). Moreover, treatment of CD14+ cells with AG490, a commercially available inhibitor of JAK2/Stat signaling did not abrogate the induction of SOCS-3 RNA following stimulation with IL-25 and LPS/PGN (not shown). SOCS-3 expression can be also regulated by Stat-independent mechanisms. For example, SOCS-3 can be induced by MAPK in multiple cell types. So, we next determined whether MAP family members are involved in the IL-25-mediated SOCS-3 induction. This possibility was first investigated using three MAPK inhibitors. Notably, pre-treatment of CD14+ cells with SB202190, an inhibitor of p38, abrogated the inducing effect of LPS and IL-25 on SOCS-3 RNA expression (FIG. 12(a)). By contrast, both PD98059, an inhibitor of ERK, and 420116, an inhibitor of JNK, did not block the IL-25-mediated induction of SOCS-3 RNA following LPS stimulation (FIG. 21). Similarly, SB202190, but neither PD98059 nor 420116 abrogated the inhibitory effect of IL-25 on cytokine response following PGN stimulation (FIG. 22).

To directly test whether IL-25 activates p38 MAPK, total extracts from CD14+ cells cultured in the presence or absence of IL-25 were analyzed for the content of phosphorylated (p)-p38. Data in FIG. 12(b) show that IL-25 enhanced the level of p-p38 in a time-dependent fashion. Moreover, pre-incubation ofCD14+ cells with IL-25 augmented the expression of p-p38 in LPS- (FIG. 12(c)) and PGN- (not shown) treated cells. Specificity of SB202190 was confirmed by showing that this compound blocked p38 but neither JNK nor ERK1/2 phosphorylation in LPS+IL-25-treated cells (FIG. 12(d)). Consistent with these data, SB202190 abrogated the negative effect of IL-25 on the cytokine expression induced by LPS and PGN (FIG. 12(e), and not shown). Finally, we showed that SB202190 did not alter the rate of CD14+ cell apoptosis (3,3 % vs 3,6 % in cells cultured with DMSO), thus excluding the possibility that its effect on the IL-25-mediated SOCS-3 induction was a reflection of nonspecific toxicity. From these experiments, we concluded that activation of p38 is required for optimal induction of SOCS-3 by IL-25 in human CD14+ cells.

### IL-25-treated mice exhibit decreased in vivo cytokine response

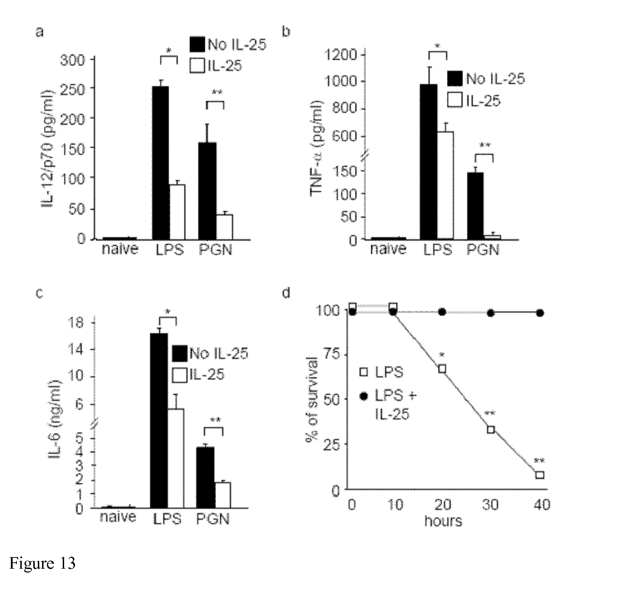

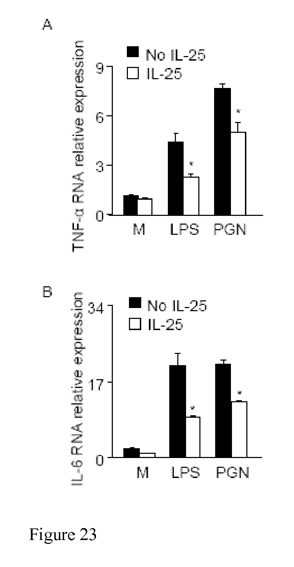

The in vitro studies described above provided considerable evidence that IL-25 negatively regulates multiple inflammatory pathways in human monocytes. To confirm this notion in vivo, we initially determined whether IL-25 inhibited the cytokine response induced by systemic administration of LPS and PGN. Before conducting these experiments, we examined whether IL-25 modulated the LPS/PGN responses in mouse as in the case of human monocytes. For this, CD11c cells were isolated from spleens of mice, pre-incubated with murine IL-25 for 30 minutes and then stimulated with LPS/PGN. As shown in FIG. 23, IL-25 significantly inhibited the expression of TNF and IL-6 induced by LPS and PGN. Subsequently, we measured the serum level ofIL-12p70, TNF-α and IL-6 in mice following intraperitoneally injection of LPS or PGN and/or IL-25. In both cases, IL-25 was given intraperitoneally 1 h before LPS/PGN, and serum samples collected after 5 hours. As expected, the serum levels ofIL-12/p70, TNF-α, and IL-6 were markedly increased by systemic administration of LPS or PGN (FIGs. 13 (a)-(c)). Mice pre-treated with IL-25 exhibited diminished production of all cytokines ((FIGs. 13 (a)-(c)). Since serum levels of these inflammatory cytokines positively correlate with the LPS-induced lethal endotoxemia, we next assessed whether IL-25 also increases the survival of LPS-treated mice. To this end, we administered a single dose of IL-25 or PBS (vehicle) to mice and 1 h later animals were injected with LPS. IL-25-treated mice did not die, while a time-dependent reduction of the survival was seen in mice receiving PBS (FIG. 13(d)). No protection was seen in mice treated with heat-inactivated IL-25 (not shown).

### IL-25 protects mice from the development of PGN-induced colitis

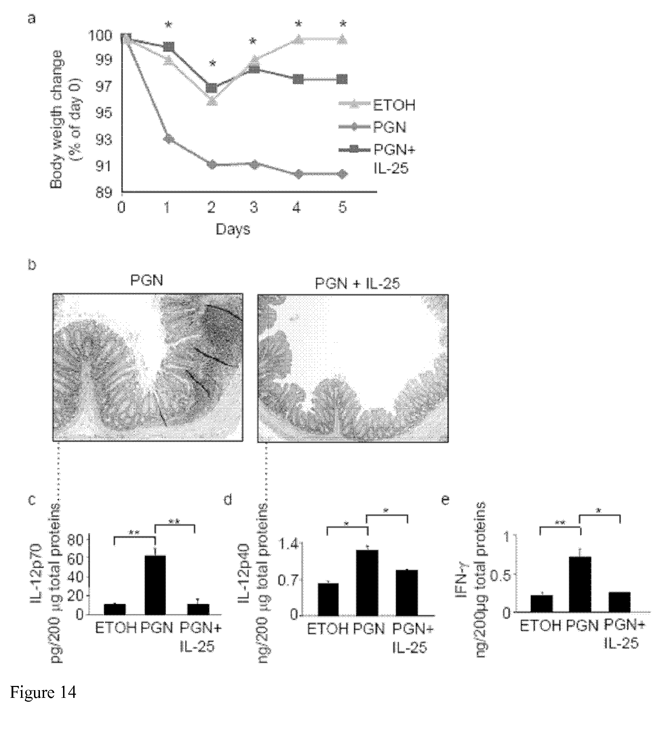

The demonstration that IL-25 reduces PGN-induced inflammatory cytokine production *in vitro* prompted us to explore the possibility that IL-25 could inhibit PGN-mediated inflammation *in vivo*. To address this issue, we used a model of colitis that is inducible in mice by rectal administration of PGN. In these studies, we administered 50% ethanol per rectum to Balb/c mice followed 8 h later by soluble PGN again per rectum. IL-25 (or PBS) was intraperitoneally injected 1 h before PGN administration. PGN-treated mice receiving no IL-25 exhibited a significant loss of body weight from the first day of PGN treatment as compared with IL-25-treated mice (FIG. 14(a), p=0.02). At day 5, these mice had lost nearly 10% of their initial body weight, had watery diarrhea, and there was 11 % mortality. By contrast, PGN-treated mice receiving IL-25 developed no diarrhea, had no significant loss of body weight as compared with ethanol-treated mice (FIG. 14(a)), and did not die. Histopathologically, PGN-treated mice showed marked infiltration of inflammatory cells, focal loss of crypts and reduction of goblet cells, sporadic ulcerations, and lymphoid aggregates. IL-25-treated mice showed mild infiltration of the mucosa with inflammatory, no loss of crypts, and no reduction of goblet cells (FIG. 14(b)). As PGN-induced colitis associates with enhanced Th1 cell response, we next examined whether the protection against PGN-colitis seen in IL-25-treated mice was associated with changes in the production of Th1 cytokines. To this end, total proteins were extracted from colonic specimens of mice with PGN-colitis receiving either PBS or IL-25 and controls (ethanol-treated mice) and analyzed for the content ofIL-12p70, IL-12p40, and IFN-γ by ELISA. As expected, induction of PGN-colitis was associated with a marked increase in the production ofIL-12 (both p70 and p40) and IFN-γ as compared with control mice (FIGs. 14 (c)-(e)). Notably, administration of IL-25 to mice suppressed the induction of these cytokines ((FIGs. 14 (c)-(e)).

### IL-25 attenuates inflammation in mice with PGN-induced and TNBS-induced colitis

In subsequent studies we determined whether IL-25 is also therapeutic in the experimental colitis. In these studies, IL-25 or PBS was administered the day after PGN injection. As indicated above, PGN-treated mice receiving no IL-25 lost weight over the time course (FIG. 15(a)). By contrast, PGN-treated mice regained rapidly their weight after IL-25 treatment; thus the average weight of mice in the group administered IL-25 was similar to that in the group not administered PGN (ethanol-treated mice) (FIG. 15(a)). Macroscopically, the colon appeared markedly shorter in PGNtreated mice receiving no IL-25 than in IL-25-administered animals (not shown). These changes correlated with the histopathological appearance of the bowel wall of mice in the two groups (FIG. 15(b)). Consistently, the colonic levels ofIL-12p70, IL-12p40, and IFN-γ were significantly lower in mice treated with IL-25 than in animals receiving no IL-25 (FIGs. 15 (c)-(e)).

To confirm further the therapeutic properties of IL-25, we used another model of colitis that is induced by rectal administration of trinitrobenzene sulfonic acid (TNBS) and is associated with a pathogenic Th1 cell response. In these studies, IL-25 was administered the day after the induction of TNBS-cofitis. TNBS-treated mice exhibited loss of body weight independently of IL-25 treatment (not shown). However, there was 37% mortality in mice receiving no IL-25, while animals treated with IL-25 did not die. Histological examination of colon tissue as well as blinded histologic scoring of colitis in the different groups weresignificantly reduced in IL-25-treated mice as compared to untreated mice (FIG. 15(f), p=0.03).

Consistent with the above observations, the production of functionally active (heterodimeric) p70 and monomeric p40 subunit ofIL-12 and of IFN-γ were significantly increased in the colons of mice with TNBS-colitis in comparison to ethanol-treated controls (FIGs. 15 (g)-(i)). Administration of IL-25 to mice with TNBS-colitis inhibited the production of IL-12 and IFN-γ (FIGs. 15 (g)-(i)).

In a final set of experiments, we assessed whether the therapeutic effect of IL-25 was associated with a diminished ability of mucosal cells to produce inflammatory cytokines in response to TLR signals. To this end, LPMC were isolated from the colon of mice with PGN- and TNBScolitis and receiving IL-25 or PBS the day after the induction of colitis. LPMC were cultured in the presence of PGN or LPS for 6 hours, and then the RNA transcripts for IL-12 (p40 and p35), IL-6, and TNF-α were evaluated by real-time PCR. LPMC isolated from mice with either PGN- (FIG. 16 (a)) or TNBS- (FIG. 16 (b))-colitis and treated with IL-25 exhibited a significantly reduced cytokine response to LPS and PGN as compared with cells isolated from animals treated with PBS.

Overall these findings confirm that *in vivo* administration of IL-25 results in a marked inhibition of the cytokine response induced by both PGN and LPS.

### DISCUSSION

In this study we show that human blood and intestinal CD14+ cells express high levels of IL-25R, and that IL-25 negatively regulates CD14+ cell inflammatory cytokine expression driven by various inflammatory stimuli, including TLR ligands (i.e., PGN, LPS). Analysis of molecular mechanisms underlying such an inhibitory effect revealed that IL-25 does not alter the expression of both TLR-2 and TLR-4, and does not interfere with TLR signaling, as neither induction of costimulatory molecules not the phagocytosis of fluorescent beads by CD14+ cells following LPS/PGN stimulation were significantly affected by IL-25. Since IL-25 has no effect on the expression of IL-10 induced by LPS/PGN and did not increase the fraction of AV/PI-positive cells, it is unlikely that inhibition of TLR responses by IL-25 is due to induction of either cell exhaustion of cytokine production or cell death. IL-25 inhibited also the expression of cytokines induced by IFN-γ and TNF-γ, thus indicating that it makes CD14+ cells resistant to multiple inflammatory signals rather than blocking specific signaling pathways.

Remarkably, the expression of a previously identified inhibitory molecule of TLR and cytokine receptor signals, namely SOCS-3, was enhanced by IL-25 in CD14+ cells following stimulation with LPS or PGN. Subsequent studies strongly suggested that SOCS-3 was the mediator of the IL-25-induced inhibition of CD14+ cell cytokine response. Indeed, silencing of SOCS-3 gene expression by specific siRNA abolished the inhibitory effect of IL-25.

Another novel and interesting observation uncovered by our study is that induction of SOCS-3 by IL-25 relies on the activity of p38 MAPK. Corroborating previous results, we found that IL-25 by itself is sufficient to trigger p38 phosphorylation, and to cooperate with both LPS and PGN in enhancing the level of active p38 in CD14+ cells. Moreover, it was shown that preincubation of CD14+ cells with a specific inhibitor ofp38, but not with inhibitors of ERK or JNK, completely abolished the IL-25-mediated SOCS-3 induction. In regard to the latter findings, it has been previously reported that SOCS-3 can be induced in a cell type-specific manner by a large number of stimuli via the activation of multiple intracellular pathways, including p38 MAPK. For instance, SOCS-3 expression can be enhanced by activation of p38 in B cells following stimulation with IL-4, and in macrophages following stimulation with LPS, CpG, and TNF-α, or infection with Mycobacterium avium. There is also evidence that, in hepatocytes, IL-6-induced SOCS-3 expression occurs via the p38 MAPK pathway. The molecular basis underlying the regulation of the IL-25-driven SOCS-3 induction by p38 MAPK is still obscure. A possibility is that IL-25 regulates SOCS-3 at the transcriptional level. Indeed, previous studies have shown that SOCS-3 promoter contains numerous potential regulatory elements, and that activation of p38 signaling cascade in HepG2 cells enhances basal activity or IL-6-induced transcriptional activation of a SOCS3 promoter reporter construct (Bode JG, 1: Biol Chem. 2001 Oct;382(10):1447-53). Transcriptional activation of the SOCS3 promoter by IL-6 requires specificity protein 3 (Sp3), (Ehlting C, Biochem J. 2005 May 1;387(Pt 3):737-45), a ubiquitously expressed transcription factor that can be activated by p38 (Xu k, 1: Cancer Res. 2007 Jul 1;67(13):6121-9.). Another and more convincing hypothesis is that IL-25 may enhance SOCS-3 mRNA stability by activating p38. In this context, it is noteworthy that p38 has been demonstrated to be necessary for TNF-α-mediated stabilization of SOCS-3 mRNA in macrophages, and that both fibroblasts and macrophages deficient for MAPK-activated protein kinase 2, a downstream target of p38, have reduced levels of SOCS-3 in part due to a diminished SOCS-3 mRNA stability. Studies are now in progress to address these issues, as well as to examine the molecular mechanisms upstream of p38 MAPK activation in IL-25-treated CD 14+ cells.

We further strengthened the importance of IL-25 in the negative control of cytokine response by CD14+ cells in a well-established model of endotoxemia. In particular, administration of IL-25 to mice exerted immunomodulatory effects on LPS- and PGN-driven production of IL-12, IL-6, and TNF-α. These cytokines are believed to play a pivotal role in LPS-induced endotoxic shock, and inhibition of the LPS-induced cascade of pro-inflammatory cytokines is the primary mechanism through which anti-inflammatory molecules confer protection against the lethal effects of LPS administration. In line with this, we showed that IL-25 treatment protected animals from the LPS-induced lethality. Unfortunately we could not confirm the specificity of the IL-25-mediated protection against LPS-induced shock because there is no commercially available antibody capable of blocking IL-25. As an alternative approach we however showed that heat-inactivated IL-25 did not protect mice from LPS-induced lethality, thus supporting the specificity of the immunoregulatory effects of IL-25.

Further in vivo studies showed that administration of IL-25 to mice led to the amelioration of both PGN- and TNBS-induced colitis, and that this effect was paralleled by a marked inhibition of the production of inflammatory cytokines in the gut. More importantly, the counter-regulatory effect of IL-25 was seen regardless of whether the cytokine was administered before or after the induction of colitis. We cannot exclude the possibility that the efficacy of IL-25 treatment on the ongoing colitis is in part dependent on the broader effects of this cytokine within the intestinal microenvironment. In fact, IL-25 is known to antagonize Th17 cell responses that have been recently shown to play a decisive role in the pathogenesis of colitis. Determination of which of these immunoregulatory effects of IL-25 prevail in the models of colitis here described will require careful experimentation to resolve. Nonetheless, our findings support previous studies showing that cytokines capable of antagonizing the production of pro-inflammatory mediators can be effective in mitigating mucosal inflammation. At the same time, our data suggest that IL-25 may be an attractive candidate for the treatment of colitis.

### MATERIALS & METHODS

### Cell isolation and culture

All reagents were from Sigma-Aldrich (Milan, Italy) unless specified. Human peripheral blood mononuclear cells (PBMC) were isolated from enriched buffy coats of healthy volunteer donors and patients with IBD by Ficoll gradients, and used to assess IL-25R or to purify CD14+ cells by using CD14 magnetic beads (Miltenyi Biotec, Bologna, Italy). Cell purity was routinely evaluated by flow cytometry and ranged between 94 and 98%. An aliquot of CD14+ cells was used to extract RNA, while the remaining cells were resuspended in RPMI 1640 medium, supplemented with 10% inactivated fetal bovine serum (FBS), penicillin (100 U/mL), and streptomycin (100 µg/mL) (Life Technologies-GibcoCRL, Milan, Italy), and seeded in 48-well culture dishes (1x10⁶ cells/well). CD14+ cells were incubated with IL-25 (50 ng/ml, R&D Systems, Inc. Minneapolis, MN, USA) for 30 minutes and then stimulated with LPS (100 ng/ml) or PGN (10µg/ml), or TNF-α (R&D Systems), or IFN-γ (50 ng/ml, Peprotech EC LTD, London, UK) for 30 minutes to 48 hours. At the end, cells were used to extract RNA or to analyze cell-surface protein by flow-cytometry, while cell-free supernatants were used for ELISA. To examine the effect of IL-25 on p38 MAPk and Stat3, CD14+ cells were stimulated with IL-25 (50 ng/ml) for 5-30 minutes, then lysed and total extracts analyzed for the content ofp-p38, total p38, p-Stat3, and total Stat3 by Western blotting. In parallel, cells were pre-incubated with IL-25 for 30 minutes and then stimulated with LPS/PGN for further 15-30 minutes. To assess the role of MAPK and Stat molecules on the IL-25-induced SOCS-3 expression, cells were pre-incubated with PD98059 (50µM), an inhibitor of ERK1/2, or SB202190 (10µM), an inhibitor of p38, or 420116 (5µM), a JNK inhibitor, or AG490 (100 µM), a Jak2/Stat inhibitor (all from Inalco, Milan, Italy), or DMSO (vehicle) for 30 minutes prior to adding IL-25 and/or LPS/PGN. Moreover, cells were pre-incubated with SB202190 or DMSO, then treated with or without IL-25 (30 minutes) and finally stimulated with LPS/PGN for 6 hours. At the end, cells were used to analyze cytokine by real-time PCR. To examine the role of SOCS-3 in the IL-25-mediated negative regulation of CD 14+ cell cytokine response, CD 14+ cells were cultured with or without human SOCS-3 or control siRNA according to the manufacturer instructions (Santa Cruz Biotechnology, Santa Cruz, CA). After 48 hours, cells were washed and cultured with or without IL-25 for 30 minutes and then stimulated with LPS or PGN for further 2-6 hours.

### IBD samples and Lamina propria mononuclear cell isolation and culture

Colonic specimens were taken from 9 patients with CD and 6 patients with UC undergoing surgery for a chronic active disease poorly responsive to medical treatment. At the time of surgery, 7 patients with CD and 4 patients with UC were receiving corticosteroids, while the remaining CD patients and UC patients were receiving immunosuppressive drugs and corticosteroids. Lamina propria mononuclear cells (LPMC) were prepared by using the DTT-EDTA-collagenase procedure, and used for assessing IL-25R or purifying CD14+ cells by positive MACS separation. The resulting cell preparations contained >95% CD14+ cells as assessed by flow cytometry. Cells were resuspended in complete RPMI 1640 medium, and cultured with or without IL-25 (50 ng/ml) for 30 minutes and then stimulated with LPS. After 6 hours, cells were harvested, and used for extracting RNA.

### Flow cytometry

IL-25R was evaluated in freshly obtained blood samples, total PBMC, total LPMC, and purified blood and intestinal CD 14+ cells by using the following monoclonal anti-human antibodies: CD56 FITC (1:10 final dilution, Diaclone Research, Milan, Italy), CD3 PerCP (1:50, final dilution, Becton Dickinson, Milan, Italy), CD14 FITC (1:50 final dilution, Immunotools, Friesoythe; Germany), CD16 FITC (1:50 final dilution, Immunotools), CD19 (1:50 final dilution, Immunotools), isotype control IgGs (Becton Dickinson), IL-25R PE (1:10 final dilution, R&D Systems). To evaluate cell activation markers, CD14+ cells were stained with the following antibodies: anti-human TLR4 PE or TLR2 PE (both at 1:5 final dilution, eBioscience, San Diego, CA, USA), anti-human HLD-DRII PE or anti-human CD86 FITC (both at 1:50 final dilution, Becton Dickinson) or anti-human CD80 APC (1:50 final dilution, Immunotools). Incubations were carried out at 4°C for 30 minutes. Cells were then washed, resuspended in PBS and analyzed by flow-cytometry.

To analyze whether IL-25 affects the phagocytic properties of CD 14+ cells, CD 14+ cells were seeded in a 6-well dishes and preincubated with IL-25 (50 ng/ml) for 30 minutes and then stimulated with LPS (100 ng/ml) or PGN (10 µg/ml) in presence of orange fluorescent latex beads (5x10⁶ beads/ml) for 6 hours at 37°C. Cells were then washed, resuspended in PBS and analyzed by flow-cytometry.

To assess cell apoptosis, cells were cultured as indicated, and the percentage of Annexin V (AV) and/or propidium iodide (PI)-positive cells was then assessed by flow cytometry (FACSCalibur, Becton Dickinson) according to the manufacturer's instructions (Immunotech, Marseille, France).

### RNA extraction, cDNA preparation, RT-PCR and Real-time PCR

RNA was extracted by TRIzol reagent according to the manufacturer's instructions (Invitrogen, Milan, Italy). A constant amount of RNA (500 ng/sample) was retro-transcribed into complementary DNA (cDNA), and 1 µl of cDNA/sample was then amplified using the following conditions: denaturation 1 minute at 95°C, annealing 30 seconds at 58°C for human IL-23p19, mouse IL-12/p40 and mouse TNF-α, at 60°C for both human and mouse IL-6, at 62°C for human TNF-α, human IL-10, human IL-25R membrane-bound isoform, and for both human and murine β-action, followed by 30 seconds of extension at 72°C. Transcripts for IL-25R soluble form were analyzed by a semi-quantitative PCR, using the following conditions: denaturation for 30 seconds at at 95°C, annealing for 30 seconds at 55°C, and extension for 1 minute at 72 °C for a total of 35 cycles. Primers sequence was as follows: human IL-23p19, FWD:5'-GGGACACATGGATCTAAGAG-3' and REV: 5'-GCAAGCAGAACTGACTGTTG-3'; human IL-6, FWD: 5'-CCACTCACCTCTTCAGAACG-3' and REV: 5'-GCCTCTTTGCTGCTTTCACAC-3'; human IL-10, FWD: 5'-GGCACCCAGTCTGAGAACAG-3' and REV: 5'-CTTGGCAACCCAGGTAACCC-3'; human TNF-α, FWD: 5'-AGGCGGTGCTTGTTCCTCAG-3' and REV: 5'-GGCTACAGGCTTGTCACTCG-3'; human IL-25R membrane-bound isoform, FWD: 5'-CCTCCGAGTAGAACCTGTTAC-3' and REV: 5'-AGTTGCTTTTGCCCGTCACAC-3', human IL-25R soluble form, FWD: 5'-AGGTGGGGACACAGGAGGTC-3' and REV: 3'-CTGGAGGACACAGGGGTGGA-5'; mouseIL-23p40, FWD: 5'-GGTTTGCCATCGTTTTGCTG-3' and REV: 5'-GAGGTTCACTGTTTCTCCAG-3'; mouse IL-6, FWD: 5'-AGCCAGAGTCCTTCAGAGAG-3' and REV: 5'-GATGGTCTTGGTCCTTAGCC-3'; mouse TNF-α, FWD: 5'-ACCCTCACACTCAGATCATC-3'and REV: 5'-GAGTAGACAAGGTACAACCC-3'. Human IL-12/p40, human IL-12/p35, human SOCS-1 and SOCS-3, mouse IL-12p35 were evaluated using a commercially available TaqMan probe (Applied Biosystems, Foster City, CA, USA). β-actin (FWD: 5'-AAGATGACCCAGATCATGTTTGAGACC-3' and REV:5'-AGCCAGTCCAGACGCAGGAT-3) was used as an internal control. Real-time PCR was performed using the IQ SYBR Green Supermix (Bio-Rad Laboratories, Milan, Italy). **Systemic administration of LPS, PGN, and IL-25**

Balb/c mice were administered intraperitoneally with recombinant mouse IL-25 (R&D Systems; 10 µg/20 gr/mouse) followed 1 h later by intra-peritoneal injection of LPS (300 µg/20 gr/mouse) or PGN (300 µg/20 gr/mouse). Serum samples were collected at 5 hours. Survival of mice was monitored over the next 7 days.

### Induction of PGN and TNBS colitis and IL-25 administration

PGN colitis was induced in Balb/c mice by intrarectal administration of 100 µL of 50% ethanol followed 8 hours later by soluble PGN (1mg/20 gr/mouse) again per rectum. Controls included mice that were given intrarectal administration with 50% ethanol but not PGN. For induction of TNBS-cofitis, 2.5 mg of TNBS in 50% ethanol was administered to lightly anesthetized mice through a 3.5F catheter inserted into the rectum. The catheter tip was inserted 4 cm proximal to the anal verge, and 150 µL of fluid was slowly instilled into the colon, after which the mouse was held in a vertical position for 30 seconds. Controls consisted of mice treated with 150 µL of 50% ethanol.

Recombinant mouse IL-25 (10µg/20 gr/mouse) was injected intra-peritoneally 1 hour before the administration of PGN, in the preventive model, whereas the cytokines was injected 1 day after the administration of PGN or TNBS, in the therapeutic model. Weight changes were recorded daily to assess the induction of colitis, and tissues were collected for histologic study, protein analysis, and isolation of LPMC. For histologic analysis, tissues were fixed in 10% neutral buffered formalin solution, embedded in paraffin, cut into tissue sections, and stained with H&E.

For TNBS-induced colitis, stained sections were examined for evidence of colitis and assigned a colitis score by considering the presence of acute and chronic inflammatory infiltrates, elongation and/or distortion of crypts, frank ulceration, and thickening of the bowel wall, as described elsewhere.

### Protein extraction, Western blotting, and cytokine ELISA

Total proteins were prepared from cell and tissue samples as previously indicated (Boirivant et al). For Western blotting analysis, the membranes were blocked with Tris-buffered saline containing 0.05% Tween 20 and 5% nonfat dry milk and then incubated with specific phosphorylated and total MAPK, phosphorylated and total Stat3 antibodies (Santa Cruz Biotechnology). Appropriate horse-radish peroxidase-conjugated secondary antibodies were then used and bound antibodies visualized using enhanced chemiluminescence (Pierce, S.I.A.L., Rome, Italy). Computer-assisted scanning densitometry (Total lab, AB.EL S.r.1., Rome, Italy) was used to analyze the intensity of the immunoreactive bands.

Total proteins extracted from mice with PGN- and TNBS colitis and controls either left untreated or treated with IL-25 were used to analyze IL-12p70, IL-12p40, and IFN-γ by commercially available ELISA kits (IL-12p70 from R&D Systems, IL-12p40 and IFN-γ from Peprotech) according to the manufacturer's instructions. Supernatants of human blood CD14+ cells were analyzed for IL-12/p40 and TNF-α by ELISA (both kits from Peprotech).

### Data analysis

Difference between groups will be compared using either the Mann-Whitney U test, if the data will not be normally distributed, or the Student's T test, if the observations will be consistent with a sample from a normally distributed population. The survival curves from experiments with systemic injection of LPS and/or IL-25 were analyzed by the Kaplan-Meier method., and Wilcoxon statistics were generated to test the homogeneity between treatment groups.

**Table 1. IL-25 inhibits the expression of inflammatory cytokines induced by LPS and PGN in human blood CD14+ cells. CD14+ cells were isolated from 5 healthy volunteers and preincubated with medium or IL-25 for 30 minutes then either left unstimulated (M= medium) or stimulated with LPS, or PGN for further 6 hours. RNA was then extracted and amplified by real-time PCR using specific primers for the indicated cytokines. Levels are normalized to β-action and indicate the mean ± SD of all experiments. LPS/PGN-treated cells vs LPS/PGN+ IL-25-treated cells: * p=0.02; **p=0.04.**

| | | medium | | LPS | | PGN | |
|---|---|---|---|---|---|---|---|
| | IL-25 | - | + | - | + | - | + |
| P40 | | 1,8±0,3 | 2,8±1,2 | 616±120 | 234±57 (*) | 629±243 | 286±130 (*) |
| P35 | | 1,9±0,67 | 4,4±2,8 | 21±7,7 | 3,4±1,4 (*) | 1,4±0,4 | 1,2±0,2 |
| P19 | | 1,5±0,3 | 1,4±0,1 | 182±47 | 71±17,9 (*) | 151,5±11 | 100±32 (**) |
| TNF | | 1,28±0,1 | 1,53±0,2 | 35±9,1 | 13±5,9 (*) | 35,2±11 | 5,72±0,97 (*) |
| IL-6 | | 3,4±1,87 | 5±2,8 | 7240±1957 | 2584±1496 (*) | 11670±368 | 2141±538 (*) |
| IL-10 | | 35,5±0,3 | 22,8±3,7 | 11935±760 | 9834±4134 | 2229±1061 | 2642±1548 |

**Table 2. IL-25 inhibits the expression of inflammatory cytokines induced by LPS in IBD mucosal CD14+ cells. CD14+ cells were isolated from the inflamed gut of patients with CD and preincubated with medium or IL-25 for 30 minutes then either left unstimulated (M= medium) or stimulated with LPS for further 6 hours. RNA was then extracted and amplified by real-time PCR using specific primers for the indicated cytokines. Levels are normalized to β-actin and indicate the mean ± SD of all experiments. * p<0.001; ** p<0.04.**

| | | medium | | LPS | |
|---|---|---|---|---|---|
| | IL-25 | - | + | - | + |
| P40 | | 3,83 ± 0.8 | 3,77 ± 2 | 46,6 ± 7 | 25 ± 4,6 (**) |
| P35 | | 0,6 ± 0.1 | 0,1 ± 0.01 | 13,5 ± 1,1 | 1,1 ± 0,6 (*) |
| P19 | | 2,3 ± 0.3 | 2,1 ± 0,2 | 518 ± 21 | 191,5 ± 27 (*) |
| TNF | | 2,2 ± 0.3 | 3,5 ± 0,4 | 10,3 ± 0,5 | 4,6 ± 0,2 (**) |
| IL-6 | | 2,1 ± 0.4 | 3,1 ± 0,3 | 41,6 ± 2 | 23,3 ± 1,04 (**) |
| IL-10 | | 1,7 ± 0.4 | 1,5 ± 0.1 | 5,6 ± 0,3 | 6,4 ± 0,4 |

### EXAMPLE 3

### IL-25 receptor (IL-25R) is expressed by human blood and mucosal CD14+ cells

Figure 10a shows representative dot-plots showing IL-25R in human PBMC. PBMC were isolated from healthy volunteers and simultaneously analyzed for the expression of IL-25R, CD3, CD14, CD19, and CD56. Numbers in quadrants indicate the percentages of CD3, CD14, CD19, and CD56 cells positive for IL-25R. One of six representative experiments in which similar results were obtained is shown. Figure 10b shows percentages of CD3, CD 14, CD19, and CD56 cells positive for IL-25R in fresh blood samples. Data indicate mean ± SD of 4 separate experiments in which blood samples taken from 4 healthy volunteers were analyzed. Figure 10c shows CD16+ cells express IL-25R. CD14+ cells were purified from PBMC and stained for IL-25R and CD16. One of 3 representative experiments is shown. Right inset shows the mean fluorescence intensity (MFI) of IL-25R expression in CD14+ cells either positive or negative for CD16. Data indicate mean ± SD of 3 separate experiments in which PBMC taken from 3 healthy volunteers were analyzed. Figure 10d shows real-time PCR data for the membrane bound IL-25R isoform RNA transcripts in blood CD 14+ (CD 14) cells and THP-1. Levels are normalized to β-action and indicate the mean ± SD of all experiments. Right inset shows a representative electrophoretic gel showing RT-PCR products for the soluble IL-25R isoform and β-actin in RNA samples prepared from human blood CD14+ cells and Top-1. Figure 10e shows representative dot-plots showing IL-25R in human LPMC isolated from one patient with CD and one patient with UC, and analyzed for the expression of IL-25R, CD3, and CD14. Numbers in quadrants indicate the percentages of cells positive for IL-25R. Staining with a non relevant istotype IgG is also shown. Two of five representative experiments, in which LPMC isolated from 3 CD patients and 2 UC patients were studied, are shown.

### SOCS3 levels in response to IL-25 stimulation of CD14+ cells induced by LPS and PGN.

Figure 11a shows SOCS3 levels in response to IL-25 stimulation in blood CD14+ cells induced by LPS and PGN. CD14+ cells were isolated from 4 healthy volunteers and pre-incubated with or without IL-25 (50 ng/ml) for 30 minutes then either left unstimulated or stimulated with LPS or PGN for the indicated time points. RNA was then extracted and amplified by real-time PCR using specific primers for SOCS-3. Levels are normalized to β-actin and indicate the mean ± SD of all experiments. (Untreated *vs* LPS-treated cells, § p=0.04; untreated *vs* PGN-treated cells, § p=0.03, §§ p=0.02; LPS-treated *vs* LPS+IL-25-treated cells * p=0.001; PGN-treated *vs* PGN+IL-25-treated cells ** p= 0.03). Figure 11b shows cells were cultured with control or SOCS-3 siRNA. After 2 days, cells were washed and cultured with IL-25 for 30 minutes followed by stimulation with LPS for 2 hours. SOCS-3 RNA expression was evaluated by real-time PCR. Levels are normalized to β-actin and indicate the mean ± SD of all experiments. LPS-treated *vs* LPS+IL-25-treated cells * p=0.001. Figure 11c shows IL-25 fails to inhibit cytokine expression induced by LPS and PGN in SOCS-3-deficient cells. Cells were treated with control or SOCS-3 siRNA. After 2 days, cells were washed and cultured with IL-25 for 30 minutes followed by stimulation with LPS or PGN for 6 hours. RNA was then extracted and amplified by real-time PCR using specific primers for the indicated cytokines. Levels are normalized to β-actin and indicate the mean ± SD of all experiments. LPS/PGN-treated *vs* LPS/PGN+IL-25-treated cells * p<0.04.

### Induction of SOCS-3 by IL-25 relies on p38 MAPK activity.

Figure 12a shows CD14+ cells were pre-incubated with SB202190 (a p38 Map kinase inhibitor), or vehicle (DMSO) then treated with IL-25 and/or LPS as indicated in materials and methods. SOCS-3 RNA was evaluated by real-time PCR. Levels are normalized to β-actin and indicate the mean ± SD of all experiments. *p=0.001. Figure 12b shows CD14+ cells were cultured with or without IL-25 for the indicated time points, and both p-p38 and total p38 were then evaluated by Western blotting of total extracts. Lower inset shows the quantitative analysis of p-p3 8/total p38 protein ratio, as measured by densitometry scanning of Western blots. Values are expressed in arbitrary units (a.u.). Figure 12c shows CD14+ cells were cultured with or without IL-25 for 30 minutes and then either left unstimulated or stimulated with LPS for the indicated time points. At the end, both p-p38 and total p38 were then evaluated by Western blotting of total extracts. Lower inset shows the quantitative analysis of p-p3 8/total p38 protein ratio, as measured by densitometry scanning of Western blots. Values are expressed in arbitrary units (a.u.). Figure 12d shows pre-incubation of cells with SB202190 prevents the phosphorylation of p38, but not that of both JNK and ERK1/2, in response to IL-25 and LPS stimulation. Cell culture and Western blots were performed as indicated in material and methods. One of 4 representative experiments in which similar experiments were obtained is shown. Figure 12e shows pre-incubation of cells with SB202190 prevents the negative regulation of IL-20 on the cytokine response induced by LPS. Cells were cultured as indicated in material and methods, and RNA was then extracted and analyzed for the indicated cytokines by real-time PCR. Levels are normalized to β-actin and indicate the mean ± SD of all experiments.

### Effects of IL-25 serum cytokine levels

Figure 13 shows that IL-25-treated mice exhibit decreased serum levels ofIL-12p70 (a), TNF-α (b), and IL-6 (c) following systemic administration of LPS or PGN. Groups of 5 Balb/c mice were injected intraperitoneally with PBS or PBS containing IL-25 (10 µg/20gr/mouse). One hour later animals received PBS (naive), LPS (300 µg/mouse) or PGN (300 µg/20gr/mouse). Serum samples were collected at 5 hours and analyzed for cytokine levels by ELISA: Data indicate the mean ± SD of all experiments. * p=0.03; ** p=0.02. Figure 13d shows that IL-25 protects mice from lethality. Mice were injected with or without IL-25 and then treated with LPS as indicated above. Survival was monitored for the indicated time points. Similar results were obtained in 3 separate experiments. * p=0.02; ** p=0.001.

### IL-25 prevents the induction of PGN-mediated colitis.

Figure 14a shows PGN colitis was induced in Balb/c mice by intrarectal administration of 100 µL of 50% ethanol followed 8 hours later by soluble PGN again per rectum. Controls included mice that were given intrarectal administration of 50% ethanol (ETOH) but not PGN. Recombinant mouse IL-25 was injected intra-peritoneally 1 hour before the administration of PGN, and body weight was daily recorded. Data indicate the cumulative mean weight data from 2 separate experiments. In each experiment, each group consisted of at least five mice. PGN *vs* PGN+IL-25, * p= 0.02. Figure 14b shows histologic evaluation of PGN colitis in mice treated or not with IL-25. Photomicrograph (x 40) of an H&E-stained paraffin section of a representative colon from mice belonging to each group. Severe mucosal mononuclear cell infiltrate and distortion of the normal crypt architecture with epithelial ulceration and loss of goblet cells is evident in the colon of mice with PGN receiving no IL-25. In contrast, only small scattered areas of cellular infiltration is seen in the colon of mice treated with IL-25. The photomicrographs are representative of 2 separate experiments in which at least 5 mice per group were studied. Figure 14 (c-e) shows administration of IL-25 in mice with PGN-colitis results in decreased tissue expression of IL-12p70 (c), IL-12p40 (d), and IFN-γ (e). One hour before the PGN-colitis induction, mice were either left untreated or treated with IL-25 (10 µg/mouse). Mice were then sacrificed at day 5, and colonic samples used for extracting total proteins. Cytokines were analyzed by ELISA. Data are expressed as pg/200 µg total proteins, and indicate SD of 3 separate experiments (* p=0.03; ** p=0.01).

### IL-25 ameliorates the PGN-mediated colitis.

Figure 15 shows that PGN colitis was induced in Balb/c mice by intrarectal administration of 100 µl of 50% ethanol followed 8 hours later by soluble PGN again per rectum. Controls included mice that were given intrarectal administration of 50% ethanol (ETOH) but not PGN. Recombinant mouse IL-25 was injected intra-peritoneally the day after the induction of colitis. Figure 15a shows body weight changes of control and colitic mice either untreated or treated with IL-25 were daily recorded. Data indicate the cumulative mean weight data from 2 separate experiments. In each experiment, each group consisted of at least four mice. PGN-treated *vs* PGN+IL-25-treated mice * p=0.02. Figure 15b shows representative H&E stained sections of colon collected from mice with PGN-colitis either left untreated or treated with IL-25. Induction of PGN-cofitis is evidenced by a marked infiltration of the mucosa with inflammatory cells, reduction of goblet cells, epithelial ulceration, and extensive destruction of mucosal layer. By contrast, IL-25-treated mice show a mild mucosal infiltration of inflammatory cells and minimal reduction of goblet cells. Figure 15(c-e) shows Administration of IL-25 in mice with PGN-colitis results in decreased tissue expression ofIL-12p70 (c), IL-12p40 (d), and IFN-γ (e). IL-25 was injected to mice with PGN-colitis as indicated in (a) Mice were then sacrificed at day 5, and colonic samples used for extracting total proteins. Cytokines were analyzed by ELISA. Data are expressed as pg/200 µg total proteins and indicate ±SD of 3 separate experiments (* p=0.01). Figure 15f shows IL-25 ameliorates the TNBS-mediated colitis. TNBS colitis was induced in Balb/c mice by intrarectal administration of 2.5 mg of TNBS in 50% ethanol. Controls included mice that were given intrarectal administration of 50% ethanol (ETOH) but not TNBS. Recombinant mouse IL-25 was injected intra-peritoneally the day after the induction of colitis. Histologic evaluation of TNBS-cofitis in mice treated or not with IL-25. Photomicrograph (x40) of an H&E-stained paraffin section of a representative colon from mice belonging to each group. Severe mucosal mononuclear cell infiltrate and disruption of the normal crypt architecture with epithelial ulceration and loss of goblet cells is evident in the colon of mice with TNBS. In contrast, only a minimal cellular infiltration of the mucosa is seen in the colon of mice treated with IL-25. The photomicrographs are representative of 3 separate experiments in which at least 5 mice per group were studied. Right insets. Histologic score of the colon sections taken from control (ETOH) and colitic mice either treated with or without IL-25. Data indicate mean SD of 3 separate experiments. For each experiment at least 5 mice per group were considered. * p=0.01. Figure 15 (g-i) shows Administration of IL-25 in mice with TNBS-colitis results in decreased tissue expression of IL-12p70 (g), IL-12p40 (h), and IFN-γ (i). IL-25 was injected as indicated in a, and mice were then sacrificed at day 5. Cytokines were analyzed by ELISA of colonic extracts. Data are expressed as pg/200 µg total proteins and indicate ±SD of 3 separate experiments. * p=0.01.

### IL-25 administration reduces the cytokine response of LPMC from mice with PGN- and TNBS-colitis to both LPS and PGN.

Figure 16 shows Colon LPMC which were isolated from mice with PGN- (a) or TNBS- (b) colitis, either receiving PBS or IL-25 the day after the induction of colitis, on day 4, and stimulated in vitro with PGN or LPS for 6 hours. RNA expression for IL12p40, IL12p35, TNF-α, and IL-6, was evaluated by real-time PCR. Levels are normalized to β-actin and indicate the mean ± SD of all experiments. * p=0.03; **p=0.04.

### TLR-2 and TLR-4 in blood CD14+ cells

Figure 17 shows representative dot-plots of TLR-2 and TLR-4 in blood CD14+ cells either left unstimulated (Uns) or stimulated with 50 ng/ml IL-25 in the presence or absence of PGN (a) or LPS (b) for 6 hours. Cells were simultaneously analyzed for the expression of CD14 and TLR-2 or TLR-4. Numbers in quadrants indicate the percentage of cells in the designated gates. One of four separate experiments is shown. Right inset show the mean fluorescence intensity of TLR-2 and TLR-4 respectively in cells stimulated as indicated above.

### CD80, CD80, and HLA-DRII in blood CD14+ cells

Figure 3 shows representative dot-plots showing CD80, CD80, and HLA-DRII in blood CD14+ cells either left unstimulated (Uns) or stimulated with 50 ng/ml IL-25 for 30 minutes followed by treatment with or without LPS or PGN for 48 hours. Cells were simultaneously analyzed for the expression of CD80 and CD86 or CD80 and HLA-DRII. Numbers in quadrants indicate the percentage of cells in the designated gates. One of two separate experiments is shown.

### Effects of IL-25 on the ability of LPS-stimulated human blood CD14+ cells to phagocytosi.

Figure 18 shows that IL-25 does not alter the ability of LPS-stimulated human blood CD14+ cells to phagocyte fluorescent latex beads. Cells were preincubated with IL-25 (50 ng/ml) for 30 minutes and then stimulated with LPS in presence of orange fluorescent latex beads. After 6 hours, cells were analyzed by flow-cytometry. Numbers in quadrants indicate the percentage of cells in the designated gates. One of four separate experiments is shown.

### Effects of IL-25 on the expression of inflammatory cytokines induced by TNF-α and IFN-γ in human blood CD14+ cells.

Figure 19 shows that IL-25 inhibits the expression of inflammatory cytokines induced by TNF-α and IFN-γ in human blood CD14+ cells. CD14+ cells were isolated from healthy volunteers and preincubated with or without IL-25 (50 ng/ml) for 30 minutes then either left unstimulated (M= medium) or stimulated with TNF-α or IFN-γ for further 6 hours. Cytokine RNA was analyzed by real-time PCR. Levels are normalized to β-action and indicate the mean ± SD of all experiments. * p=0.003; ** p=0.04.

### SOCS-1 RNA levels in response to IL-25 stimulation

Figure 20 shows that SOCS-1 RNA levels in response to IL-25 stimulation in blood CD14+ cells are induced by LPS and PGN. CD14+ cells were isolated from 4 healthy volunteers and pre-incubated with or without IL-25 (50 ng/ml) for 30 minutes then either left unstimulated or stimulated with LPS or PGN for the indicated time points. SOCS-1 RNA was then analyzed by real-time PCR. Levels are normalized to β-action and indicate the mean ± SD of all experiments. unstimulated *vs* LPS or PGN stimulated cells § p=0.03; LPS-treated *vs* LPS+IL-25-treated cells * p=0.01.

### Assessment of JNK/ERK activity in the induction of SOCS-3 by IL-25

Figure 21 shows that induction of SOCS-3 by IL-25 does not rely on JNK/ERK activity. CD14+ cells were preincubated with 420116 (a JNK inhibitor), PD9805 (an ERK inhibitor), or vehicle (DMSO) then treated with IL-25 and/or LPS as indicated in materials and methods. SOCS-3 RNA was evaluated by real-time PCR. Levels are normalized to β-action and indicate the mean ± SD of all experiments.

### Assessment of p38 Map kinase activity in the induction of SOCS-3 by IL-25

Figure 22 shows that induction of SOCS-3 by IL-25 relies on p38 Map kinase activity. CD14+ cells were preincubated with SB202190 (a p38 Map kinase inhibitor) (panel a), 420116 (a JNK inhibitor) (panel b), PD9805 (an ERK inhibitor) (panel b), or vehicle (DMSO) then treated with IL-25 and/or PGN as indicated in materials and methods. SOCS-3 RNA was evaluated by real-time PCR. Levels are normalized to β-action and indicate the mean ± SD of all experiments. *p=0.001.

### Effects of IL-25 on TNF-α and IL-6 induced by LPS and PGN in CD14+ cells

Figure 23 shows that IL-25 inhibits the expression of TNF-α (A) and IL-6 (B) induced by LPS and PGN in CD14+ cells isolated from the spleen of Balb/c mice. CD14+ cells were preincubated with medium (no IL-25) or IL-25 (50 ng/ml) for 30 minutes then either left unstimulated (M= medium) or stimulated with LPS or PGN for further 6 hours. RNA was then extracted and amplified by realtime PCR. Levels are normalized to β-action and indicate the mean ± SD of all experiments. * p=0.01.

Having thus described several aspects of at least one embodiment of this invention, it is to be appreciated various alterations, modifications, and improvements will readily occur to those skilled in the art. Such alterations, modifications, and improvements are intended to be part of this disclosure, and are intended to be within the spirit and scope of the invention. Accordingly, the foregoing description and drawings are by way of example only. All references described herein are incorporated by reference for the purposes described herein.

Moreover, this invention is not limited in its application to the details of construction and the arrangement of components set forth in the disclosed description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

## Claims

1. The use of interleukin-25, a derivative of or a fragment of, in the manufacture of a medicament for the treatment of diseases, resulting from cytokine production by CD14+ cells.

2. A use according to claim 1, wherein the disease is an inflammatory disease.

3. A use according to claim 2, wherein the disease is selected from the group comprising, Inflammatory bowel disease (IBD), Crohn's disease (CD), ulcerative colitis, coronary artery disease, sepsis, endotoxemia, arthritis rheumatoid, immune-complex induced and proliferative glomerulonephritis, tubulointerstitial nephritis, vasculitis, viral and autoimmune hepatitis, autoantibody-mediated diseases, psoriasis, neoplasia and other CD14+ cell related diseases.

4. The use according to any preceding claim involving down regulating the production of inflammatory cytokines.

5. A use according to claim 4, wherein the inflammatory cytokines are involved in a TH1 and/or TH-17 response.

6. A use according to claim 5, wherein the inflammatory cytokines are IL-6 and IL-8, TNF-α, IL12, IL-23, IL-22, IL-21 IFN-gamma, IL-17 and IL-1β.

7. A pharmaceutical composition containing a pharmaceutically active amount of interleukin-25, a derivative thereof or a fragment of IL-25, together with a pharmaceutically acceptable carrier or excipient.

8. A method of down regulating the inflammatory cytokine response of CD 14+ cells comprising administering of interleukin-25, a derivative or a fragment thereof.

9. An antibody targeting IL-25R, for use in the manufacture of a medicament for the treatment of CD14+ associated diseases.

10. The use of interleukin-25, a derivative of or a fragment of, as described herein with reference to the examples.

11. A pharmaceutical composition substantially as described herein with reference to the examples.

12. A method of down regulating the inflammatory cytokine response substantially as described herein with reference to the examples.

13. An antibody substantially as described herein with reference to the examples.

14. The use of interleukin-25, a derivative of or a fragment of, in the manufacture of a medicament for the treatment of diseases, known to result from cytokine production by CD 14+ cells.
